# EUROPEAN PATENT SPECIFICATION

(11) **EP 1 581 560 B1**
(45) Date of publication and mention of the grant of the patent: **04.05.2011**
(21) Application number: 03784106.1
(22) Date of filing: 28.07.2003
(51) Int. Cl.: C07K 16/42, A61P 7/04, A61K 39/395, A61K 38/37, C07K 16/36

(54) **ANTI-IDIOTYPIC ANTIBODIES AGAINST FACTOR VIII INHIBITOR AND USES THEREOF**
ANTI-IDIOTYPISCHE ANTIKÖRPER GEGEN FAKTOR-VIII-HEMMER UND DEREN VERWENDUNGEN
ANTICORPS ANTI-IDIOTYPIQUES DIRIGÉS CONTRE UN INHIBITEUR DU FACTEUR VIII ET LEURS UTILISATIONS

(30) Priority: 31.07.2002 EP 02447150
(43) Date of publication of application: 05.10.2005
(73) Proprietor: Life Sciences Research Partners, 3000 Leuven (BE)
(72) Inventor: GILLES, Jean-Guy, G., B-1090 Bruxelles (BE); SAINT-REMY, Jean-Marie, R., B-1390 Grez-Doiceau (BE); JACQUEMIN, Marc, G., B-5330 Sart-Bernard (BE)
(74) Representative: Bird, Ariane
(86) International application number: PCT/EP2003/008365
(87) International publication number: WO 2004/014955

(56) References cited:
- WO-A-99/58680
- JACQUEMIN M G ET AL: "MECHANISM AND KINETICS OF FACTOR VIII INACTIVATION: STUDY WITH AN IGG4 MONOCLONAL ANTIBODY DERIVED FROM A HEMOPHILIA A PATIENT WITH INHIBITOR" BLOOD, W.B. SAUNDERS, PHILADELPHIA, VA, US, vol. 92, no. 2, 1998, pages 496-506, XP000906844 ISSN: 0006-4971 cited in the application
- SAINT-REMY J-M R ET AL: "ANTI-IDIOTYPIC ANTIBODIES: FROM REGULATION TO THERAPY OF FACTOR VIII INHIBITORS" VOX SANGUINIS, S. KARGER AG, BASEL, CH, vol. 77, no. SUPPL 1, 1999, pages 21-24, XP008012175 ISSN: 0042-9007
- GILLES JEAN GUY G ET AL: "Antibodies to idiotypes of human monoclonal anti-factor VIII (FVIII) antibodies neutralise their inhibitory activity." BLOOD, vol. 94, no. 10 SUPPL. 1 PART 1, 15 November 1999 (1999-11-15), page 460a XP001109476 Forty-first Annual Meeting of the American Society of Hematology;New Orleans, Louisiana, USA; December 3-7, 1999 ISSN: 0006-4971 cited in the application
- LUBAHN B C ET AL: "Characterization of a monoclonal anti-idiotype antibody to human anti-factor VIII antibodies." PROCEEDINGS OF THE NATIONAL ACADEMY OF SCIENCES OF THE UNITED STATES OF AMERICA. UNITED STATES NOV 1990, vol. 87, no. 21, November 1990 (1990-11), pages 8232-8236, XP002227743 ISSN: 0027-8424 cited in the application
- GILLES JEAN GUY ET AL: "Neutralizing antiidiotypic antibodies to factor VIII inhibitors after desensitization in patients with hemophilia A." JOURNAL OF CLINICAL INVESTIGATION, vol. 97, no. 6, 1996, pages 1382-1388, XP002227744 ISSN: 0021-9738 cited in the application
- SULTAN Y.; ROSSI F.; KAZATCHKINE D.: 'Recovery from anti-VIII:C (antihemophilic factor) autoimmune disease is dependent on generation of antiidiotypes against anti-VIII:C autoantibodies' PROCEDURES OF THE NATIONAL ACADEMY OF SCIENCES OF THE UNITED STATES OF AMERICA vol. 84, February 1987, pages 828 - 831

## Description

### FIELD OF THE INVENTION

The present invention relates to pharmaceutical compositions for use in the treatment of haemophilia, especially for use in the treatment of human patients who have developed FVIII inhibitors directed to the C2 domain. The present invention provides anti-idiotypic antibodies against FVIII inhibitors directed to the C2 domain. The invention further relates to monoclonal cell lines expressing such anti-idiotypic antibodies. The invention also relates to pharmaceutical compositions comprising the anti-idiotypic antibodies of the present invention.

### BACKGROUND OF THE INVENTION

Haemophilia A is an X-linked disorder characterised by the absence or insufficient amount of functional factor VIII, a 330kD glycoprotein molecule produced by the liver as a single polypeptide chain of 2332 amino acids. This deficiency affects 1 in 10,000 males and can result in uncontrolled bleeding in joints, muscles and soft tissues. Patients affected by the severe form of the disease (FVIII activity lower than 1% of normal level) suffer from spontaneous bleedings. Patients with corresponding FVIII activity from 1 to 5%, or higher than 5% are defined as moderate or mild haemophilia A, respectively, and suffer from limited bleeding occurring after minor trauma or surgery. The coagulation pathway can be restored by administration of FVIII concentrates prepared from plasma or produced by recombinant cDNA technology.

The human FVIII gene has been isolated and expressed in mammalian cells, as reported by various authors, including Wood et al. in Nature (1984) 312: 330-337 and the amino-acid sequence was deduced from cDNA. U.S. Patent No. 4,965,199 discloses a recombinant DNA method for producing FVIII in mammalian host cells and purification of human FVIII. The human FVIII detailed structure has been extensively investigated. The cDNA nucleotide sequence encoding human FVIII and predicted amino-acid sequence have been disclosed for instance in U.S. Patent No. 5,663,060. In a FVIII molecule, a domain may be defined as a continuous sequence of amino-acids that is defined by internal amino-acid sequence homology and sites of proteolytic cleavage by a suitable protease such as thrombin. The FVIII proteins has been described to consist of different domains, which for the human amino-acid sequence correspond to: A1, residues 1-372; A2, residues 373-740; B, residues 741-1648; A3, residues 1690-2019; C1, residues 2020-2172; C2, residues 2173-2332. The remaining sequence, residues 1649-1689, is usually referred to as the FVIII light chain activation peptide. FVIII is produced as a single polypeptide chain which, upon processing within the cell, is rapidly cleaved after secretion to form a heterodimer made of a heavy chain containing the A1, A2 and B domains and a light chain made of the A3-C1-C2 domains, according to Kaufman et al. (1988, J Biol Chem 263:6352-6362). The two chains are non-covalently bound by divalent cations. Both the single-chain polypeptide and the heterodimer circulate in plasma as inactive precursors, as taught by Ganz et al. (1988, Eur J Biochem 170:521-528). Activation of factor VIII in plasma initiates by thrombin cleavage between the A2 and B domains, which releases the B domain and results in a heavy chain consisting of the A1 and A2 domains, according to Eaton et al. (1986, Biochemistry 25:505-512). Human recombinant FVIII may be produced by genetic recombination in mammalian cells such as CHO (Chinese Hamster Ovary) cells, BHK (Baby Hamster Kidney) cells or other equivalent cells.

Pratt et al. (1999, Nature 402:439-42) disclose the detailed structure of the carboxy-terminal C2 domain of human FVIII, which contains sites that are essential for its binding to von Willebrand factor (vWF) and to negatively charged phospholipid surfaces. This structure, which reveals a beta-sandwich core from which two beta-turns and a loop display a group of solvent-exposed hydrophobic residues, partly explains mutations in the C2 region that lead to bleeding disorders in haemophilia A. According to Gale et al. (2000, Thromb. Haemost. 83:78-85), of the at least 250 missense mutations that cause FVIII deficiency and haemophilia A, 34 are in the C domains.

FVIII is a cofactor of the intrinsic pathway of the coagulation cascade, which acts by increasing the proteolytic activity of activated factor IX over factor X, in the so-called tenase complex formation. Patients suffering from haemophilia A present bleedings which are either spontaneous in the severe form of the disease, or occur after trauma in the mild/moderate forms.

Haemophilia A patients are usually treated by replacement therapy, which consists in infusing human FVIII either purified from pools of donor plasma, or obtained by cDNA recombination technology.

The majority of the patients are immunologically unresponsive to these infusions, but for yet unclear reasons, 25% of them mount an IgG immune response towards FVIII, which can result in complete inhibition of the procoagulant activity of infused FVIII (Briët E et al. in (1994) Throm. Haemost.; 72: 162-164 ; Ehrenforth S et al. in (1992) Lancet; 339:594). Such specific IgG, which belong to the IgG 1, 2, 4 subclasses, are called FVIII inhibitors. Published studies have demonstrated that the anti-FVIII immune response is polyclonal, and primarily directed towards the A2, A3 and C2 domains (Scandella D et al. in (1989) Blood; 74: 1618-1626.; Gilles JG et al. in (1993) Blood; 82: 2452-2461).

Recent studies using human monoclonal antibodies derived from the peripheral memory B cell repertoire of inhibitor patients indicated that important epitopes are also located on the C1 domain (Jacquemin M et al. in (2000) Blood 95:156-163). The mechanism by which anti-FVIII antibodies interfere with the function of FVIII are numerous, including proteolytic cleavage of FVIII and interaction with different partners such as von Willebrand factor (vWF), phospholipids (PL), FIX, FXa or APC. Most of these mechanisms are now well described in studies using mouse or human anti-FVIII antibodies. Thus, antibodies can reduce the rate at which FVIII is activated by either binding to a proteolytic cleavage site or by inducing a 3D conformational change in FVIII that renders it less amenable to proteolysis. Antibodies interfering with the binding of vWF to FVIII appear to be very efficient as inhibitors, as shown in recent studies using human monoclonal antibodies directed towards the C2 domain, which is one of the major vWF binding sites (Jacquemin M et al in (1998) Blood; 92:496-501). Suppressing the production of inhibitors and establishing a state of immune unresponsiveness to FVIII remains a major goal. The medical community is, however, far from reaching these goals, due basically to the limited understanding of the mechanisms underlying specific antibody production and regulation.

Presently, to control such an immune response, several treatments are used including bypassing agents such as desmopressin (DDAVP), agents promoting coagulation such as prothrombin complex concentrates (PCC) or activated PCC, recombinant FVIIa, plasmapheresis and infusions of large or intermediate doses of FVIII (200-300 IU/kg body weight or 25-50 IU/kg body weight, respectively). However, none of these methods are satisfactory and they are all very costly.

Based on these observations and on the understanding of the mechanisms of immune tolerance, anti-idiotypic antibodies appear to be a promising way of treating inhibitors. In fact, it is well established that tolerance to self-protein is first induced at an early stage by clonal deletion of self-reactive B and T cells in the bone marrow and the thymus, respectively. However, not all self-reactive lymphocytes are eliminated by central deletion. Auto-reactive B cells are a common feature of peripheral blood, as well as low- or intermediate-affinity self-reactive T cells. A number of mechanisms by which such auto-reactive cells are rendered non-functioning or are deleted in the periphery have been described. Anti-idiotypic antibodies can represent a third level of tolerance maintenance in this general scheme, with their capacity to fine tune the function of antibodies and maintain a subtle equilibrium between complementary idiotypes expressed on B and T cells.

A good indication of how anti-idiotypic antibodies can exert a regulatory mechanism in the periphery is provided by the demonstration that healthy individuals with normal levels of FVIII produce significant titres of inhibitory antibodies to FVIII (Algiman M et al. in (1992) Proc Natl Acad Sci USA 89 :3795-3799; Gilles JG et al in (1994) J Clin Invest 94:1496-505), the activity of which is undetectable in plasma because of the presence of complementary anti-idiotypic antibodies. However, such a FVIII inhibitory activity can be readily detected when anti-FVIII antibodies are purified by a combination of chromatography and specific immunoadsorption over insolubilized FVIII. The FVIII inhibitory capacity of affinity-purified antibodies was demonstrated to be equal to that of anti-FVIII antibodies purified from haemophilia A patient's plasma with high level of inhibitors, as measured by Bethesda assay (Gilles JG et al. in (1994) J Clin Invest 94:1496-505).

Such neutralising anti-idiotypic activity has also been detected in a group of patients successfully desensitised by administration of high doses of FVIII (Gilles JG et al. in (1996) J Clin Invest 97:1382-1388). The study demonstrated that the concentration of anti-FVIII antibodies, purified by the same procedure as for healthy donors, did not change during desensitisation and that antibodies maintained their capacity to inhibit the procoagulant function of FVIII, even though the titration of inhibitor using the Bethesda assay in plasma was reduced to undetectable levels. This pointed to the potentially important function of anti-idiotypic regulation in tolerance to FVIII molecule. Therefore, any novel therapy inducing an increased production of anti-idiotypic antibodies can be of interest in the treatment of patients with FVIII inhibitors. A first approach along these lines has been reported, in which patients were treated with injections of immune complexes made of FVIII and autologous specific antibodies towards FVIII, which resulted in a significant reduction in the level of circulating FVIII inhibitors, which were neutralised by corresponding anti-idiotypic antibodies (Gilles JG, Arnout J. XXI International Congress of the World Federation of Haemophilia 1994 April ; abstract). Such an approach can open the way towards new therapeutic strategies, which target FVIII inhibitors at potentially low cost compared to presently available treatments.

Previous findings from the inventors' laboratory have shown that anti-idiotypic antibodies exert physiological properties in the homeostasis of the anti-FVIII immune response. Thus, the peripheral blood of healthy individuals contains antibodies specific for FVIII, some of which have the property of inhibiting the procoagulant function of FVIII (Gilles JGG & Saint-Remy JMR in (1994) J Clin Invest 94: 1496-1505). In these individuals, the function of FVIII is actually not altered, as antibody-mediated FVIII inhibition is neutralised by specific anti-idiotypic antibodies. We therefore concluded that anti-idiotypic antibodies have a physiological relevance in the maintenance of normal FVIII activity.

Moreover, the inventors have shown that when haemophilia A patients with inhibitor are treated by regular infusion of high doses of FVIII, a treatment also called desensitisation or tolerance induction (see above), one of the biological consequences of such infusions is the elicitation of specific anti-idiotypic antibodies able to neutralise the inhibitor (Gilles JG et al. in (1996) J Clin Invest 97: 1382-1388). These findings suggest that the use of anti-idiotypic antibodies could represent a valuable approach for the control of FVIII inhibitory antibodies.

The human monoclonal antibody B02C11 is a FVIII-specific IgG4kappa antibody derived from the natural repertoire of a patient with inhibitor (Jacquemin MG, et al. in (1998) Blood 92: 496-506). Ab BO2C11 recognises the C2 domain and inhibits the binding of FVIII to both vWF and phospholipids (PL). This antibody is representative of a major class of human inhibitory antibodies. Its mechanism of action is commonly encountered in patients with inhibitor and C2-specific antibodies are the most frequently observed inhibitory antibodies. Moreover, the exact binding site of Ab B02C11 on the C2 domain has been deciphered through X-ray analysis of crystals made of the antibody Fab fragments and the C2 domain (Spiegel P.C. Jr. et al. (2001) Blood 98: 13-19).

Previous reports have described anti-idiotypic antibodies directed towards anti-FVIII antibodies. Sultan et al (1987) Proc Natl. Acad. Sci USA 84 824-831, describes a polyclonal anti-idiotypic F(AB')2 fragment obtained from a patient who has successfully overcome auto-antibody mediated inhibition of FVIII activity and which inhibits anti FVIII:C activity of autoantibodies. Lubahn and Reisner ((1990), Proc Natl Acad Sci. USA 87: 8232-8236) describe partially purified human anti-FVIII antibodies by salt precipitation and chromatography from the plasma of a haemophilia A patient with inhibitor. These authors demonstrated that the purified IgG recognised the native FVIII heavy chain and its thrombin-digested 43kDa chain. The preparation (SP8.4) was injected in mice in an attempt to obtain anti-idiotypic antibodies. Several clones were obtained, with one of them, Mab20-2H, inhibiting the anti-FVIII antibody binding to the heavy chain. In a functional assay, Mab20-2H did not modify the inhibitory activity of SP8.4 IgG fraction, even when added at high concentrations. Mab20-2H detected antibodies in 3.2% of the haemophilic inhibitor plasmas tested, but never neutralised the inhibitory activity. The authors concluded that Mab20-2H recognised a non-inhibitory anti-FVIII antibody that is directed toward the 43kDa chain (A2 domain) of the FVIII molecule.

Another anti-idiotypic antibody (B6A2C1) was developed and described from the inventors' laboratory (Gilles JG et al in (1999) Blood 94, abstract 2048: 460a). This anti-idiotypic antibody is directed towards an anti-FVIII C1 domain inhibitor.

### SUMMARY OF THE INVENTION

It is an object of the present invention to provide a pharmaceutical composition for use on human patients suffering from (primary or acquired) haemophilia, who have developed inhibitors against FVIII.

It is a further object of the present invention to provide cell lines for generating neutralising antibodies directed against human FVIII inhibitors as well as the neutralising antibodies.
One aspect of the invention relates to a monoclonal anti-idiotypic antibody against a human Factor VIII inhibitory antibody, the said inhibitory antibody being directed towards the C2 domain of Factor VIII, characterized by the fact that the complementary determining regions of the variable heavy chains of said anti-idiotypic antibody have the amino acid sequences depicted in SEQ ID NO:5, SEQ ID NO:6, and SEQ ID NO:7, respectively and the complementary determining regions of the variable light chains of said antibody have the amino acid sequences depicted in SEQ ID NO:8 SEQ ID NO:9 and SEQ ID NO:10, respectively and by the fact that the anti-idiotypic antibody a) neutralises the anti-coagulant activity of said human Factor VIII inhibitory antibody for at least 50% in a chromogenic FVIII assay and b) does not interfere with the binding of FVIII to vWF and phospholipids.

According to one embodiment the anti-idiotypic antibody is directed against a FVIII inhibitory antibody of which the heavy chain is encoded by a VH germline segment DP-5 derived from the VH1 family. In yet another embodiment of the present invention the Factor VIII inhibitory antibody is Ab BO2C11.

Other embodiments of the present invention relate to humanised anti-idiotypic antibodies.

Further embodiments relate to monoclonal anti-idiotypic antibodies, such as the one obtainable from cell line 14C12. The cell line 14C12 was deposited on July 30, 2002 at the Belgian Coordinated Collections of Micro-organisms (BCCM), LMBP (plasmid collection, Laboratorium voor Moleculaire Biologie, Universiteit, K.L. Ledeganckstraat 35, 9000 Gent, Belgium) with Accession Number LMBP 5878CB.

Further embodiments are anti-idiotypic antibodies wherein the variable heavy chain of anti-idiotypic antibody is encoded by the nucleotide sequence depicted in SEQ ID NO: 1 or a nucleotide sequence having at least 70 % sequence identity, preferably having at least 80 % sequence identity, more preferably having at least 90% sequence identity, even more preferably having at least 95-99 % sequence identity with SEQ ID NO: 1, and wherein the variable light chain of the anti-idiotypic antibody is encoded by the nucleotide sequence depicted in SEQ ID NO: 3 or a nucleotide sequence having at least 70 % sequence identity, preferably having at least 80% sequence identity, more preferably having at least 90% sequence identity, even more preferably having at least 95-99 % sequence identity with SEQ ID NO: 3. In particular, the nucleotide sequences may include those which use equivalent trinucleotides to those specified in the SEQ ID No 1 and SEQ ID NO: 3 as determined by the redundancy of the genetic code, thereby retaining the ability to neutralise inhibitory FVIII antibodies.

Further embodiments relate to anti-idiotypic antibodies having a variable heavy chain protein sequence as depicted in SEQ ID NO: 2 or a protein sequence having at least 70 % sequence identity, more preferably at least 80 % sequence identity, even more preferably having at least 90% sequence identity, still even more preferably having at least 95% sequence identity, most preferably having at least 99 % sequence identity with SEQ ID NO: 2 and/or having a variable light chain protein sequence as depicted in SEQ ID NO: 4 or a protein sequence having at least 70 % sequence identity, more preferably at least 80 % sequence identity, even more preferably having at least 90% sequence identity, still even more preferably having at least 95% sequence identity, most preferably having at least 99% sequence identity with SEQ ID NO: 4, thereby retaining the ability to neutralise inhibitory FVIII antibodies.

Other embodiments relate to modified versions of the anti-idiotypic antibodies of the present inventions and also relates to F(Ab')2 fragments, Fab' fragments, Fab fragments and other fragments comprising at least one CDR region of an anti-idiotypic antibody directed against an antibody against the C2 domain of FVIII. The invention also relates to modified versions of said fragments.

Yet another aspect of the present invention is a monoclonal cell line expressing an anti-idiotypic antibody such as the deposited monoclonal cell line 14C12 with Accession Number LMBP 5878CB.

Another aspect of the present invention is a pharmaceutical composition comprising the above described anti-idiotypic antibodies against inhibitory antibodies against the C2 domain of Factor VIII and fragments, and modified versions thereof, such as disclosed in details herein above.

Another aspect of the present invention is the use of the above described anti-idiotypic antibody or fragments, and modified versions thereof as a medicine.

Another aspect of the present invention is the use of the above described anti-idiotypic antibody or fragments, and modified versions thereof for the manufacture of a medicament for the prevention or treatment of bleeding, more particularly of bleeding in a haemophilia patient with inhibitory antibody against the C2 domain of FVIII.

Another aspect of the present invention is the use of the above described anti-idiotypic antibody or fragments, said anti-idiotypic antibody and modified versions thereof for the manufacture of a medicament for inhibition of production of inhibitory antibodies. According to one embodiment of this aspect of the invention, the above described anti-idiotypic antibodies directed against FVIII inhibitors are used for the induction of apoptosis of B cells carrying anti C2 inhibitory antibodies. Thus the anti-idiotypic antibodies and their fragments of the present invention are capable of suppressing both the effector function of inhibitors to the C2 domain of FVIII (neutralisation of the effect of soluble antibodies) and the production of such antibodies (interactions with cells carrying identical or similar idiotopes as B02C11).

The present invention also relates to a method of preventing or treating the bleeding in patient, optionally a haemophilia patient, having inhibitory antibodies against the C2 domain of FVIII, the method comprising the step of administering to the patient the above described anti-idiotypic antibody, fragments, or modified versions thereof.

The method also relates to a method of inducing apoptosis of B cells carrying anti-C2 inhibitory antibodies in a haemophilia patient with the inhibitory antibody against the C2 domain of FVIII of the invention or fragments said anti-idiotypic antibody and modified versions thereof.

Another aspect of the invention relates to the use of the anti-idiotypic antibodies directed against FVIII inhibitors of the invention for the detection of FVIII antibodies in body fluids.

Throughout the Description, reference is made to the following sequences represented in the sequence listing:
- SEQ ID NO: 1:: nucleotide sequence of the heavy chain variable region of Ab 14C12
- SEQ ID NO: 2:: amino acid sequence of the heavy chain variable region of Ab 14C12
- SEQ ID NO: 3:: nucleotide sequence of the light chain variable region of Ab 14C12
- SEQ ID NO: 4:: amino acid sequence of the light chain variable region of Ab 14C12
- SEQ ID NO: 5:: amino acid sequence of CDR1 (H1) of the heavy chain variable region of Ab 14C12
- SEQ ID NO: 6:: amino acid sequence of CDR2 (H2) of the heavy chain variable region of Ab 14C12
- SEQ ID NO: 7:: amino acid sequence of CDR3 (H3) of the heavy chain variable region of Ab 14C12
- SEQ ID NO: 8:: amino acid sequence of CDR1 (L1) of the light chain variable region of Ab 14C12
- SEQ ID NO:9 :: amino acid sequence of CDR2 (L2) of the light chain variable region of Ab 14C12
- SEQ ID NO:10 :: amino acid sequence of CDR3 (L3) of the light chain variable region of Ab 14C12

### DEFINITIONS

**Factor VIII** abbreviated as **FVIII** refers to the glycosylated blood coagulation factor with a molecular weight of 330kD and 2332 amino acids which undergoes proteolytic processing.

**Light chain of FVIII** refers the proteolytic processed part of FVIII containing the A3, C1 and C2 domain.

**$C2 domain** refers to the region spanning residues 2173 to 2332 of FVIII.

**Phospholipid (PL) binding site** refers to a region in the C2 domain between amino acids 2302 and 2332.

**Idiotope** refers to a single antigenic determinant.

**Idiotype** refers to the collection of idiotopes within the variable region that confers on an immunoglobulin molecule an antigenic individuality and is characteristic of a given antibody in an individual.

An Idiotype is said to correspond to the **Internal image of an epitope** when it conformationally mimics an antigenic epitope.

The term "**Antibody**" ("**Ab**") as used herein refers to intact monoclonal or polyclonal antibody molecules as well as fragments thereof comprising:
a) either both heavy and light chains, (such as Fab, F(ab)₂, F(ab')₂) or single heavy or light chains (e.g. light chain dimers), optionally including their constant region (or parts thereof), or optionally minor modifications (such as allotypic variants) of that constant region;
b) parts, thereof, in particular the specificity-determining parts thereof, i.e. the variable regions of the antibodies
c) subparts thereof, in particular the hypervariable parts thereof, such as peptides made up of stretches of amino acids comprising all six CDR sequences, optionally with adjacent framework sequences, e.g. of up to about 10 amino acid sequences at one or more CDRs.

Optionally, according to the present invention, antibodies are IgG antibodies, particularly IgG1. **F(ab')2** refers to the antibody fragment obtainable after pepsin cleavage and is built up of both light chains and parts of the heavy chains disulfide linked via the hinge region. The **Fab** fragment is obtainable from the intact antibody or from the F(ab')2 by papain digestion of the hinge region and contains a one light chain and one part of the heavy chain. Fragments of antibodies can also be obtained by synthesis or by recombinant methods described in the art.

An **"inhibitory antibody"** or "FVIII inhibitor" (Ab1) as used herein refers to an antibody which inhibits the activity of FVIII. According to the present invention, an inhibitory antibody is an antibody directed against the C2 domain of FVIII. Inhibitory antibodies can be either alloantibodies against exogenous FVIII or autoantibodies. Inhibitory antibodies can be of human or animal origin. Optionally, an inhibitory antibody is a human inhibitory antibody against human Factor VIII.

**Anti-idiotypic antibody** in the present invention refers to a second generation antibody (Ab2), which can be polyclonal but is preferably monoclonal and directed towards the variable part of inhibitory antibodies (Ab1). The anti-idiotypic antibodies according to the present invention preferably have the ability to neutralise inhibitory antibodies and optionally Ab1 production. The anti-idiotypic antibody of the present invention can be of human origin. The anti-idiotypic antibody of the present invention is preferably monoclonal and optionally recombinant.

The **"ability to neutralise inhibitory antibodies"** refers to a property of an anti-idiotypic antibody of the present invention to block the inhibitory activity of FVIII inhibitors (Ab1). This is can be determined by assaying the FVIII activity in the presence of inhibitor and anti-idiotypic Ab in an assay such as the Factor VIII chromogen test as described in Jacquemin et al. (1998) Blood 92, 494-506.

**Complementarity determining regions (CDR)** in the present invention refers to the hypervariable amino acid sequences within antibody variable regions which interact with the epitope on the antigen. In the present invention the CDR regions are the CDR1, CDR2 and CDR3 regions of the variable light (VL) and heavy (VH) chains respectively (L1, L2, L3 and H1, H2, H3 respectively) of an anti-idiotypic antibody directed against an anti C2 domain inhibitory antibody. A specific embodiment relates to the corresponding regions in the anti-idiotypic antibody 14C12.

**"Humanized antibody"** as used herein, refers to non-human antibody molecules in which amino acids have been replaced in the non-antigen binding regions in order to more closely resemble a human antibody.

A **"Reshaped human antibody"** or a **"Human hybrid antibody"** as used herein, refers to a human antibody in which amino acids in the antigen binding regions have been replaced with sequences in accordance with the present invention, e. g. CDR's, or other parts of variable regions which have been derived from the repertoire of human antibodies.

**Sequence comparisons**. Comparisons of protein or nucleotide sequences are designated in terms of sequence identity or sequence similarity. Where in accordance with the present invention comparisons are made between amino acid sequences of two VH regions or of two VL regions , or comparisons are made between two nucleotide sequences encoding CDR regions, the level of sequence identity or similarity between two sequences may include having at least 70%, preferably at least 80% more preferably at least 90%, even more preferably at least 95% and most preferably at least 99% sequence identity or similarity between two sequences.

Nucleotide or amino acid sequences which are "identical" means that when two sequences are aligned, the percent sequence identity, i.e. the number of positions with identical nucleotides or amino acids divided by the number of nucleotides or amino acids (when of different length, in the shorter) of the sequences, is higher than 70%, preferably at least 80%, even more preferably at least 90%, most preferably at least 95-99%, more specifically is 100%. The alignment of two nucleotide sequences is performed by the algorithm as described by Wilbur and Lipmann (1983, Proc Natl Acad Sci USA 80:726) using a window size of 20 nucleotides, a word length of 4 nucleotides, and a gap penalty of 4.

Two amino acids are considered as "similar" if they belong to one of the following groups GASTCP; VILM; YWF; DEQN; KHR. Thus, sequences which are similar means that when the two protein sequences are aligned the percent sequence similarity is i.e. the number of positions with identical or similar nucleotides or amino acids divided by the number of nucleotides or amino acids (when of different length, in the shorter) of the sequences, is higher than 70%, preferably at least 80%, even more preferably at least 90% and most preferably at least 95-99%, more specifically is 100%.

Nucleotide sequences are considered as "similar" if they encode the similar or identical amino acid sequences, (i.e. between two nucleotide sequences encoding identical proteins the variation is within the bounds of genetic code degeneracy). **"modified"** denotes any protein (or polypeptide) molecule in which a single or a number of amino-acids have been either substituted by any other amino-acid residue or deleted. Such amino-acid substitution or deletion can be located anywhere in the protein molecule. It also denotes protein molecules in which amino-acid residues have been substituted and/or deleted at more than a single location. In the latter case, any combination of substitution and deletion can be considered. It also refers to polymorphisms (i.e. the regular and simultaneous occurrence in a single interbreeding population of two or more alleles of a gene, where the frequency of the rarer alleles is greater, typically greater than 1%, than can be explained by recurrent mutation alone.)

### DETAILED DESCRIPTION OF THE INVENTION

The present invention will be described with reference to certain embodiments and to certain figures but the present invention is not limited thereto but only by the claims.

The present invention relates to anti-idiotypic antibodies against human Factor VIII inhibitory antibodies. Inhibitory antibodies against FVIII are most commonly developed in hemophiliacs, where the incidence of developing an inhibitor is 15-20%. Particularly haemophiliacs who are heavily exposed to FVIII concentrates, such as young patients or patients with a genetic defect that results in the absence of synthesis and/or secretion of FVIII protein (congenital hemophilia), are at high risk of developing FVIII inhibitors. However, inhibitory antibodies also occur in patients in the setting of auto-immune disorders, malignancies (such as lymphoproliferative disorders, lymphomas and solid tumors), drug reactions (e.g. penicillin, chloramphenicol, phenytoin), during pregnancy and in the post-partum state. Exceptionally, the presence of inhibitory antibodies can be idiopathic (eg in elderly patients). However, usually, the presence of inhibitory antibodies is associated with symptoms such as easy bruising and uncontrolled bleeding. This is usually referred to as acquired hemophelia. Thus, the present invention relates to compositions for use in the treatment or prevention of any condition associated with or characterised by the presence of inhibitory antibodies. The presence of inhibitory antibodies in a patient can be determined by different methods including quantitation of anti-FVIII activity, ELISA for FVIII inhibitors and purification using chromatography and immunoadsorbtion (each of these methods described in Algiman et al. 1992, above).

The present invention relates to anti-idiotypic antibodies capable of neutralising the inhibitory effect of FVIII inhibitors. Inhibition of FVIII by inhibitory antibodies can occur for instance by reducing the rate at which FVIII is activated by either binding to a proteolytic cleavage site of FVIII or by inducing a 3D conformational change in FVIII that renders it less amenable to proteolysis. Inhibition can also occur when antibodies interfere with the binding of FVIII to FIX and FX.

The present invention relates to anti-idiotypic antibodies against human Factor VIII inhibitory antibodies directed against the C2 domain of FVIII by neutralising by at least 50% the inhibition of FVIII procoagulant activity of inhibitory antibodies directed against the C2 domain of FVIII. The inhibition of procoagulant activity can be measured by the Factor VIII chromogen test as described herein. According to a particular aspect of the present invention, the anti-idiotypic antibodies are directed against inhibitory antibodies such as monoclonal antibody B02C11 (IgG4kappa antibody derived from the natural repertoire of a patient with inhibitor; Jacquemin MG, et al., 1998, Blood 92: 496-506) or other antibodies with similar C2 binding properties. More particularly, the anti-idiotypic antibodies are directed against FVIII inhibitors of which the VH domains are encoded by the DP5 VH gene segment derived from the VH1 gene family. Such and other Inhibitory antibodies can be obtained from humans (i.e. from the serum of patients which have inhibitory antibodies) or can be obtained from mice, guinea pigs, horses, goats, non-human primates and other mammals by immunization with FVIII, or fragments thereof, more particularly with a fragment comprising the all or part of the C2 domain.

The present invention relates to ant-idiotypic antibodies against human Factor VIII inhibitory antibodies directed against the C2 domain of FVIII capable of neutralising by at least 50% the inhibition of FVIII procoagulant activity of inhibitory antibodies directed against the C2 domain of FVIII, but which do not inhibit interfere with the physiological activity of FVIII, more particularly the binding of FVIII to vWF or PL. Methods for identifying the influence of an anti-idiotypic antibody on the ability of FVIII to bind to vWF and/or PL are described herein. These can be used for the selection of anti-idiotypic antibodies which do not interact with the binding of FVIII to PL or vWF, according to the present invention.

Additional methods for furthering the development of anti-idiotypic antibodies not interfering with the binding of FVIII to PL or vWF include, but are not limited to, a) using as immunogen, in the development of anti-idiotypic antibodies, an inhibitory antibody which itself has been generated against a modified FVIII, not comprising the PL binding domain (Pratt et al., above) or regions important for the binding of vWF (Jacquemin et al., 2003, J Thromb Haemost 1:456-463); b) using as immunogen, an inhibitory antibody from which the idiotope(s) recognizing the PL binding site or regions important for the binding of vWF of FVIII have been removed c) analysis of the similarity between the antigen binding site of the anti-idiotypic antibody obtained and the C2 domain of FVIII and i) selection of anti-idiotypic antibodies based on the fact that the correspondence between the idiotype of the anti-idiotypic antibody and FVIII does not include the PL binding site or regions important for the binding of vWF to FVIII or ii) removal of those regions within the idiotype of the anti-idiotypic antibody which correspond to the PL binding site of C2 or to regions for the binding of vWF to FVIII. Following each of these selection procedures, care should of course be taken that the selected or modified anti-idiotypic antibodies retain their ability to neutralise inhibitory antibodies.

According to the present invention, the monoclonal anti-idiotypic antibodies can be either of human or animal origin. Accordingly, the monoclonal anti-idiotypic antibody can be obtained in different ways. Cells producing anti-idiotypic antibodies can be obtained from peripheral blood lymphocytes from (e.g. haemophilia) patients with anti-FVIII antibodies or from healthy individuals. These can be immortalized using standard techniques and can be selected based on the ability of the anti-idiotypic antibodies to neutralize inhibitory antibodies, and, optionally, on the absence of interaction of the anti-idiotypic antibody with the binding of FVIII to PL and vWF. Optionally, the monoclonal antibodies produced by each of these cell lines can be sequenced for the identification of relevant idiotypes and selection based thereon, as described above.

Alternatively, the monoclonal anti-idiotypic antibodies of the present invention can be produced by on purpose immunisation of animals, such as mice, by injecting human FVIII inhibitory antibodies (such as the inhibitory antibody BO2C11 directed against the C2 domain of FVIII) in mice and then fusing the spleen lymphocytes with a mouse myeloma cell line, followed by identifying and cloning the cell cultures producing anti-idiotypic antibodies directed against factor VIII inhibitory antibodies.

The present invention thus further provides cell lines producing monoclonal antibodies which are reactive against FVIII inhibitors, produced as described above. These cell lines can be immortalized cells of human origin, optionally can originate from the patient to be treated. Alternatively, the immortalized cells can be of animal origin (particularly rodent). A particular embodiment of the present invention is provided by the deposited cell line 14C12 Accession Number LMBP 5878CB producing the anti-idiotypic antibody 14C12.

Optionally, the monoclonal antibodies produced in animals can be humanized, for instance by associating the binding complementarity determining region (CDR) of the non-human monoclonal antibody with human framework regions -in particular the constant C region of human gene-such as disclosed by Jones et al. in Nature (1986) 321: 522 or Riechmann in Nature (1988) 332: 323.

Alternatively, the anti-idiotypic antibodies can be obtained from human or animal sera by affinity purification on anti-FVIII antibodies (which themselves can be identified by immunoadsorption over insolubilized FVIII (as demonstrated by Algiman 1994, above and Gilles 1994, above). Optionally, the C2 domain can be used for affinity purification, in order to promote selection of anti-C2 (inhibitory) antibodies.

A particular embodiment of the present invention is provided by the anti-idiotypic monoclonal antibody 14C12 and antibodies including antibody fragments derived therefrom. The present invention relates to an anti-idiotypic antibody comprising the sequence of the CDR regions as identified in SEQ ID NOs 5 to 10.

The present invention also provides fragments of any of the above anti-idiotypic antibodies against inhibitory antibodies directed against the C2 domain of FVIII, such fragments including Fab, Fab', F (ab') 2, CDR's, single variable domains as well as modified versions thereof and combinations of these fragments and modifications.

More particularly, the present invention provides fragments and modified versions of the anti-idiotypic antibodies of the present invention, in particular fragments comprising complementarity determining regions ("CDR's") of the monoclonal anti-iditioypic antibodies, obtained as described above, as weii as modified versions thereof. For instance, the invention provides antigen-binding fragments Fab, Fab' and F (ab')2 generated by proteolytic digestion of the said monoclonal antibodies using methods well known in the art, such as described by Stanworth et al., Handbook of Experimental Immunology (1978), vol. 1 chapter 8 (Blackwell Scientific Publications). Such fragments or artificial sequences, which contain the antibody binding site, may or may not have lost a number of properties of the parent antibody, such as complement activation or capacity to bind to Fc gamma receptors, however without losing their ability to inhibit inhibitory antibodies against FVIII. The present invention also includes single chain fragment variables (scFv), single variable domain fragments of the antibodies and combination of these fragments and of the above mentioned fragments. Antibodies where the respective CDR sequences are different in one arm compared to the other arm of the antibody are also within the scope of the invention.

The present invention further provides reshaped monoclonal antibodies or human hybrid monoclonal antibodies against FVIII inhibitory antibodies directed against the C2 domain of FVIII obtained from the monoclonal anti-idiotypic antibodies described above. By human hybrid monoclonal antibodies it is meant a hybrid antibody constructed from a human antibody and from variable regions in accordance with the present invention.The present invention also provides soluble or membrane anchored single-chain variable parts of the above monoclonal antibodies. These can be obtained based on methods described in the art, an example of which is provided herewith . The DNA sequences of the variable parts of human heavy and light chains are amplified in separated reactions and cloned. A fifteen amino-acid linker sequence, for instance (Gly4 Ser) 3, is inserted between VH and VL by a two-step polymerase chain reaction (PCR), for instance according to Dieffenbach and Dveksler, "PCR Primer, a laboratory manual" (1995), Cold Spring Harbour Press, Plainview, NY, USA. The resulting fragment is then inserted into a suitable vector for expression of single chain fragment variable (scFv) as a soluble or phage-displayed polypeptide. This can be achieved by methods well known to those skilled in the art, such as described by Gilliland et al., 1996, Tissue Antigens 47: 1-20. Herein dislcosed are also ligands comprising peptides representative of hypervariable regions of a monoclonal antibody which can be obtained by synthesis using an applied biosystem synthesiser, for instance a polypeptide synthesiser such as model 9050 available from Milligen (USA) or a model from a related technology, which alone or in combination with other or similar hypervariable regions will exert properties similar to that of the parent antibody. Fragments and peptides can be generated by recombinant DNA technology. Peptides with a length up to about 100 amino acid sequences can be made by synthetic peptide synthesis such as BOC (*tert* butyloxycarbonyl) or FMOC (9-fluorenylmethoxycarbonyl) synthesis and other techniques known in the art. Optionally the amino and carboxy groups can be chemically changed in order to increase the stability and lifetime of the peptide (e.g. formylation, acetylation). Alternatively the peptide can be covalently or non-covalently bound to a carrier.

According to the present invention, anti-idiotypic antibodies for use in the treatment of patients suffering from FVIII inhibitors are optimally obtained by the following procedure:
a) isolating human antibodies directed against FVIII from a patient suffering from FVIII inhibitors
b) selecting from the antibodies obtained under (a) an antibody inhibiting FVIII coagulant activity based on its binding to C2
c) immunizing an animal with the antibodies selected under (b) or the antigen-recognizing part thereof (preferably at least VH and/or VL)
d) optionally, cloning the antibodies produced in the animal under (c)
e) selecting an anti-idiotypic antibody based on 1) its ability to neutralize the inhibitory activity of FVIII inhibitors, 2) the fact that it does not interfere with binding of FVIII to vWF or PL.
f) optionally, obtaining a humanized version, or an antibody fragment of the anti-idiotypic antibody selected under (e).

The present invention further provides a pharmaceutical composition for the prevention or treatment of bleeding in a haemophilia patient having inhibitory antibodies against the C2 domain of FVIII in humans, comprising, as an active ingredient, the monoclonal anti-idiotypic antibody and fragments and modified versions such as disclosed hereinabove, in admixture with a pharmaceutically acceptable carrier. More preferably the said monoclonal antibody is a monoclonal antibody, or a fragment, modified version obtainable from the cell line 14C12 deposited with the Belgian Co-ordinated Collections of Microorganisms Accession Number LMBP 5878CB.. A ligand in accordance with the present invention may also include a synthetic polypeptide of equivalent potency. The pharmaceutical composition of the present invention should comprise a therapeutically effective amount of the said above ingredient, such as indicated hereinafter in respect to the method of treatment or prevention.

Suitable pharmaceutical carriers for use in the pharmaceutical compositions of the invention are described for instance in Remington's Pharmaceutical Sciences 16 ed. (1980) and their formulation is well known to those skilled in the art. They include any and all solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the monoclonal antibody active ingredient in the composition.

Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the monoclonal antibody active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition comprising the active ingredient may require protective coatings. The pharmaceutical form suitable for injectionable use include sterile aqueous solutions or dispersions and sterile powders for the extemporaneous preparation thereof. Typical carriers therefore include biocompatible aqueous buffers, ethanol, glycerol, propylene glycol, polyethylene glycol and mixtures thereof.

The present invention also provides the use as a medicament of a monoclonal anti-idiotypic antibody against FVIII inhibitory antibodies directed against the C2 domain of FVIII as described herein. More preferably the medicament used in the present invention is a means suitable for reducing and/or preventing and/or treating bleeding in a haemophilia patient with inhibitory antibody against the C2 domain of FVIII. The said ligand may be provided to a patient by any means well known in the art, e.g. intravenously, intraarterially, parenterally or by catheterization.

The present invention therefore provides compositions for use in a method of treatment and/or prevention for preventing and/or treating and/or reducing the bleeding in patient, more particularly in a haemophilia patient, having inhibitory antibody against the C2 domain of FVIII in a mammal, preferably a human, comprising administering to a mammal in need of such treatment or prevention or attenuation of coagulation a therapeutically effective amount of a ligand as disclosed hereinabove. Preferably the said ligand is a monoclonal antibody obtainable from cell line 14C12 or an antigen-binding fragment Fab, Fab' or F (ab') 2, or a peptide comprising the six complementarity determining region (CDR), a soluble or membrane-anchored single-chain variable part (scFv), a single variable domain or a modified version or combination of any of these elements. Optionally said method of treatment is combined with the administration to the patient of FVIII.

A therapeutically effective amount as used herein means from about 1 microgram to about 10 milligrams per kilogram of body weight, more preferably from about 10 micrograms to about 1 milligram per kilogram of body weight, even more preferably from about 1 to about 10 milligram per kilogram of body weight of the mammal to be treated. It will be appreciated that, in view of the long half-life time of most IgG antibodies, the ligands of the present invention which are monoclonal antibodies of the said class will enjoy a periodicity of treatment which participates in the comfort of the patient.

Haemophilia A patients with inhibitor frequently produce antibodies towards the FVIII C2 domain. The ligands of the present invention such as the antibody produced by the cell line 14C12 are therefore useful for the treatment of such patients, either in emergency situations, as for instance before a surgical procedure, or as a longer term treatment in which Ab 14C12 can induce an apoptosis of B cells carrying the corresponding anti-C2 antibodies.

The ligands of the present invention, such as the anti-idiotypic antibody AB 14C12 of cell line 14C12, have the capacity to neutralise in a functional coagulation assay by at least 50 %, preferably by at least 70 %, more preferably by at least 80 % and most preferably by at least 90%, the FVIII inhibitory properties of inhibitory antibodies against the C2 domain of FVIII.

The reader skilled in the art will understand that from the knowledge of the sequence of AB 14C12 and that of the crystal structure of B02C11 Fab fragment in combination with the C2 domain, several variants of Ab 14C12 can be envisioned. Thus, point mutations can be introduced in the Ab 14C12 VH region to reinforce the internal image of C2, thereby reinforcing the inhibitory capacity of Ab 14C12 on C2 binding of B02C11 and the like. Alternatively, the sequence can be altered in such a way as to increase the number of contact residues in between BO2C11 and Ab 14C12, with or without maintenance of the C2 internal image. A particular aspect of this approach is to derive a 14C12 mutant antibody which would contain contact residues recognising amino acids included in the framework regions of BO2C11. The latter belongs to the DP5 heavy chain subfamily gene segments (Jacquemin MG et al in (1998) Blood 92: 496-506) as other antibodies with similar C2 binding properties. Since the frequency of DP5 family members is very low in the human repertoire, neutralisation of the entire DP5 subfamily can be achieved using Ab 14C12, thereby neutralising all inhibitory antibodies of which the VH domains encoded by the DP5 VH gene segment derived from the Vh1 gene family (Jacquemin et al (1998) cited supra; Van den Brink et al in (2000) Blood 99, 2828-2834.

According to the general knowledge of one skilled in the art, various different synthetic peptides can be prepared from the antibody 14C12. One or more of them, which alone or in combination can be used to neutralise B02C11-like antibodies in vivo. Such peptides are derived from complementarity determining regions (CDR) of the heavy chain of the Ab 14C12 and are represented by the peptides with amino acid sequence GYTFTSSVMHWL [SEQ ID NO:5], GYINPYNDGTKYNEKFTA [SEQ ID NO:6] and SGGLLRGYWYFDV [SEQ ID NO:7]. Alternatively these peptides are derived from complementarity determining regions (CDR) of the light chain of the Ab 14C12 and are represented by the peptides with amino acid sequence RASQDITNTLH [SEQ ID NO :8], YVSQSIS [SEQ ID NO:9] and QQSTSWPYT [SEQ ID NO:10]. Thus, synthetic peptides derived from Ab 14C12 complementarity regions (CDR) are used to infuse patients with inhibitory antibodies against the C2 domain of FVIII prior to surgery. The peptides combine with inhibitory antibodies and neutralise their inhibitory capacities. alternatively, a recombinant polypeptide is made by combining the sequence of different CDRs, with appropriate linker polypeptide sequences to maintain a suitable 3-D conformation. The polypeptide is administered in patients prior to an emergency procedure such as surgery.

An additional application of the anti-idiotypic antibodies of the present invention, such as Ab 14C12, is their use to inactivate the production of FVIII inhibitors by the immune system. For instance, the anti-idiotypic antibodies of the present invention can be used to induce apoptosis of B cells carrying anti-C2 antibodies. Such B cells, as memory cells or as some antibody producing cells, express a surface antibody equivalent to its secreted form. Cross-linking of idiotypes at the B cell surface transduces a signal leading to inhibition of proliferation or apoptosis of the cell.

A further application of the anti-idiotypic antibodies of the present invention is their use in the detection and/or purification of FVIII inhibitors. Immunological detection and purification methods which can be applied in the context of the present invention are extensively described in the art.

The following Examples may be understood in conjunction with the accompanying Figures in which:
- **Figure 1:**: Dose-dependent neutralisation of the FVIII inhibition by BO2C11, neutralised by Ab 14C12 in accordance with an embodiment of the present invention.
- **Figure 2:**: Results of reconstitution experiments in FVIII-/- C57BI/6 mice in accordance with an embodiment of the present invention. Panel A displays the reconstitution with Factor VIII in the absence or presence of inhibitor Ab BO2C11; Panel B shows the dose dependent neutralisation by Ab 14C12 of B02C11-mediated FVIII inhibition. Ab B02C11 and Ab 14C12 were preincubated; in both Panel C and B, Ab14C12 administration was followed by B02C11 and FVIII.
- **Figure 3:**: Nucleotide and amino acid sequence of the heavy and light chain of anti-idiotypic antibody 14C12. The CDR regions are indicated under the amino acid sequence.
- **Figure 4:**: Neutralisation of FVIII binding to human inhibitory antibodies against the C1 domain (2E9) and the C2 domain (B02C11) by anti-Idiotypic mouse MoAb against an inhibitor to the C1 domain (B6A2C1).
- **Figure 5:**: Superposition of the C2 domain of FVIII (black) with Ab 14C12 VH region, wherein the dark grey lines represent the FVIII C2 domain, and the light grey lines represent the VH domain of 14C12.
- **Figure 6:**: Neutralization of the inhibitory activity of B02C11 on FVIII upon addition of various concentrations of the anti-idiotypic antibody Ab 14C12. FVIII activity was measured in a functional chromogenic assay.
- **Figure 7:**: Absence of inhibition of FVIII binding to phospholipids or VWF by Ab 14C12; FVIII was added to microtitration plates coated with either phosphatidylserine (panel A) or an antibody to VWF and purified VWF (panel B). The capacity of Ab 14C12 to inhibit the binding to either PL or VWF was assayed by adding increasing concentrations of Ab 14C12. Residual FVIII binding was evaluated using an antibody directed towards the heavy chain.
- **Figure 8:**: Influence of Ab 14C12 on clearance of FVIIII from circulation; FVIII- /- C57BI/6 mice were injected IV with either FVIII alone (FVIII) or Ab 14C12 followed 15 minutes later by FVIII (VIII +14C12). The clearance rate of FVIII was evaluated by assessing FVIII function at 3 and 6 hours.
- **Figure 9:**: Binding of Ab 14C12 to B-cells producing B02C11. The B02C11 lymphoblastoid cell line carrying surface antibody was incubated with Ab 14C12 (upper panel) or with a sham antibody of the same subclass (lower panel). The right shift of the curve shown in the upper panel indicates that 68% of the lymphoblastoid cell line B02C11 is recognized by Ab 14C12.

### EXAMPLES

### EXAMPLE 1: Generation of a monoclonal Ab against FVIII inhibitor B02C11.

The Factor VIII specific human Ig4kappa BO2C11 antibody was used as for the generation of anti-idiotypic antibodies in mice. The properties of BO2C11 are described in Jacquemin et al (1998) Blood 92, 496-506. The nucleotide and amino acid sequences of the variable light and heavy chain of BO2C11 are disclosed in PCT patent application WO01/04269 .

Balb/c mice were immunised in the footpad with BO2C11 emulsified in first complete and then incomplete Freund's adjuvant. After 4 such injections, mouse serums were tested in an ELISA system for the presence of antibodies recognising BO2C11, but not other anti-FVIII antibodies or unrelated antibodies of the same isotype. Splenocytes from mice producing specific anti-B02C11 antibodies were fused with a myeloma cell lines to produce B cell clones (Kohler G et al. in (1978). Eur J Immunol 8 : 82-88.). These were expanded in culture and tested for BO2C11 specificity. One clone, 14C12, effectively bound to polystyrene plates coated with BO2C11.

The cell line 14C12 was deposited on July 30, 2002 at the Belgian Coordinated Collections of Microorganisms (BCCM), LMBP (plasmid collection, Laboratorium voor Moleculaire Biologie, Universiteit, K.L. Ledeganckstraat 35, 9000 Gent, Belgium) with Accession Number LMBP 5878CB..

### EXAMPLE 2: In vitro properties of the anti-idiotypic antibody obtained from cell line 14C12.

The properties of the anti-idiotypic antibody (Ab 14C12) obtained from 14C12 cell line were examined in in vitro assay systems. Ab 14C12 was shown to bind to BO2C11 and to inhibit in a dose-dependent manner the binding of BO2C11 to its target antigen, the FVIII C2 domain. Moreover, the capacity of Ab 14C12 to neutralise the FVIII inhibitory properties of BO2C11 in a functional coagulation assay was also assessed. The chromogenic (Faktor VIII chromogen test, Dade Behring, Marburg, Germany) is based on the conversion of a colourless substrate, which is specifically cleaved by thrombin. The test system contains every reagent required to produce thrombin, except FVIII. The intensity of colour development over time is therefore proportional to the amount of FVIII added to the system. Addition of B02C11 (0.1 µg/ml) completely inhibits the FVIII-dependent conversion (0,3 IU/ml) of the substrate.BO2C11 was added to the chromogenic assay at a final concentration of 0,1 µg/ml, which was determined as the minimum concentration of BO2C11 able to inhibit 100% of FVIII (0,3 IU/ml) in the test system. Different concentrations of Ab 14C12 were preincubated with this fixed amount of BO2C11 before addition of FVIII. The mixture was then applied to the chromogenic assay.

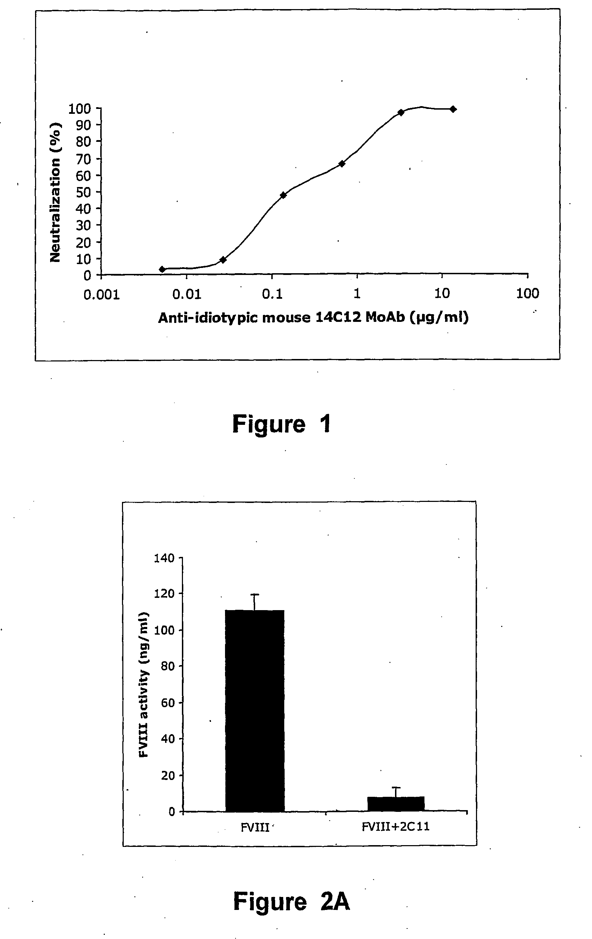

Figure 1 shows that addition of Ab 14C12 to the test system neutralises in a dose-dependent manner the inhibitory capacity of BO2C11. Moreover, 50% neutralisation is obtained at a 1/1 molar ratio with BO2C11.

The capacity of Ab 14C12 to neutralise polyclonal anti-FVIII antibodies was tested in a chromogenic assay as described above. To this end, polyclonal antibodies of the patient from whom BO2C11 was derived were added to the system as a substitute for BO2C11. Under such conditions, up to 60% neutralisation was obtained upon addition of serial dilutions of Ab 14C12. It is known that such a polyclonal antibody preparation contains a significant amount of antibodies directed towards the heavy chain of FVIII, i.e. towards epitopes fully distinct from the C2 domain. It was therefore concluded that the 60% neutralisation of inhibition observed with polyclonal antibodies is an underestimation of Ab 14C12 capacity to neutralise anti-C2 inhibitor.

In addition, Ab 14C12 significantly neutralised the inhibitory properties of polyclonal antibodies from unrelated patients. Thus, polyclonal antibodies were added at different dilutions to an immunoprecipitation assay in which radiolabelled FVIII domains are produced by combined transcription/translation using rabbit reticulocytes (Promega kit, TNT couplet reticulocyte lynate, Madison, USA). The trace amount of radiolabelled domains produced is mixed with antibody samples and protein A Sepharose. Sepharose beads are added at a concentration at which essentially all antibodies are captured. After centrifugation and washing, the radioactivity is counted on the samples, which is proportional to the presence of antibodies with specificity for the FVIII fragment tested. Thus, polyclonal antibodies of 6 unrelated patients with haemophilia A and inhibitor towards the C2 domain were added at defined concentration in an assay in which the C2 domain was translated. Ab 14C12 was then added, at increased concentration, to all samples. Three out of the 6 samples yielded significant neutralisation (circa 50%) of the inhibitory properties of polyclonal antibodies, showing that Ab 14C12 recognises an epitope commonly expressed on human anti-C2 antibodies.

### EXAMPLE 3: In vivo properties of Ab 14C12.

The capacity of Ab 14C12 to neutralise the FVIII inhibitory property of B02C11 in vivo was examined in FVIII-/- C57BI/6 mice reconstituted with human recombinant FVIII (Singh I et al. in (2002) Blood 99: 3235-3240).

Such mice are considered to be a suitable animal model for haemophilia A, insofar as no FVIII activity or antigen can be detected, with an otherwise normal phenotype (Reipert BM et al. in Thromb Haemost 2000; 84: 826-32). Administration of 1 IU human FVIII in the tail vein of FVIII-/- C57BI/6 mice resulted in a plasma concentration of 110 ng/ml after 10 min, namely ± 60% of the normal FVIII concentration in human plasma (1 IU or 192 ng/ml). When such mice are first injected with 0.5 µg of BO2C11, FVIII procoagulant activity is inhibited by 98%. The model was then used to determine whether Ab 14C12 had the capacity to neutralise the B02C11-dependent inhibition of FVIII. Thus, different concentrations of Ab 14C12 were either mixed with a fixed amount of BO2C11 (0.5 µg) before injection of the complex in FVIII-/- mice, or directly infused in mice, followed by 0.5 µg of BO2C11 and 1 IU FVIII.

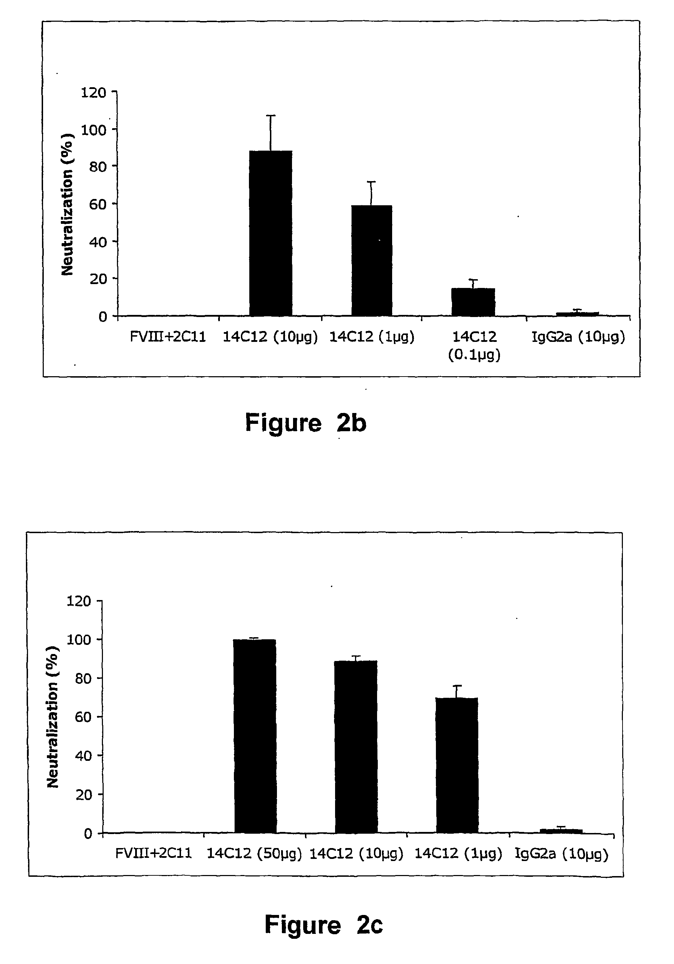

Pre-administration of 0.5 µg BO2C11 inhibited 98% of the FVIII procoagulant activity (Fig 2 A). The fixed amount of BO2C11 was preincubated with various concentrations of Ab 14C12 (0.1 to 10 µg) and the mixture infused in FVIII-/- mice. A dose-dependent neutralization of B02C11-mediated FVIII inhibition was observed (Fig 2B). The experiment was repeated but with sequential administration of Ab 14C12 followed by BO2C11 and FVIII, yielding an identical dose-dependent 14C12 mediated neutralisation (Fig 2C).

It can be seen from Figures 2A, 2B and 2C that Ab 14C12 neutralises in a dose-dependent manner the inhibition properties of BO2C11, confirming thereby the potential of Ab 14C12 as a therapy for haemophilia A patients with inhibitor towards the C2 domain of FVIII. Interestingly, as shown in in vitro assays, 50% neutralisation was obtained at an equimolar ratio between B02C11 and Ab 14C12. An Ab 14C12 molar excess of 3.5 was sufficient to fully neutralise BO2C11 and restoring a normal FVIII procoagulant activity.

### EXAMPLE 4: Binding characteristic of Ab 14C12 to BO2C11.

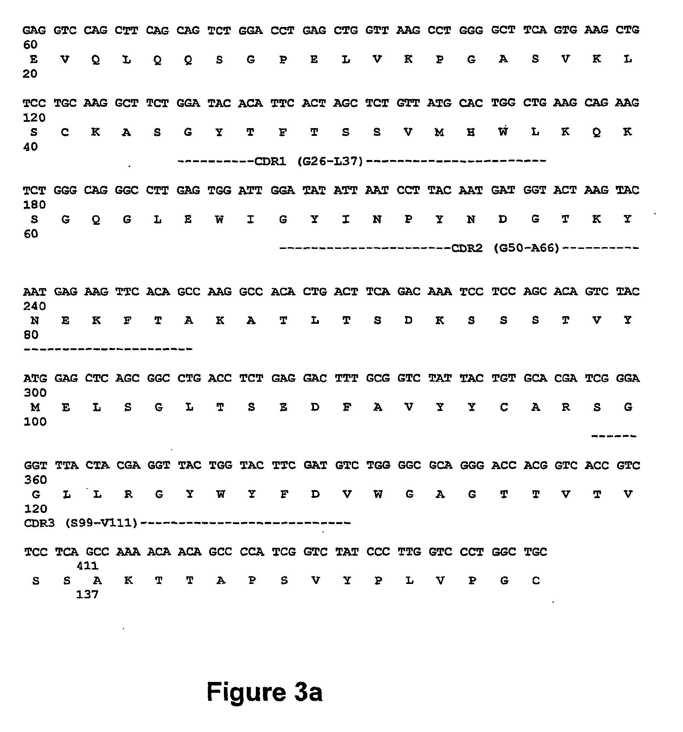

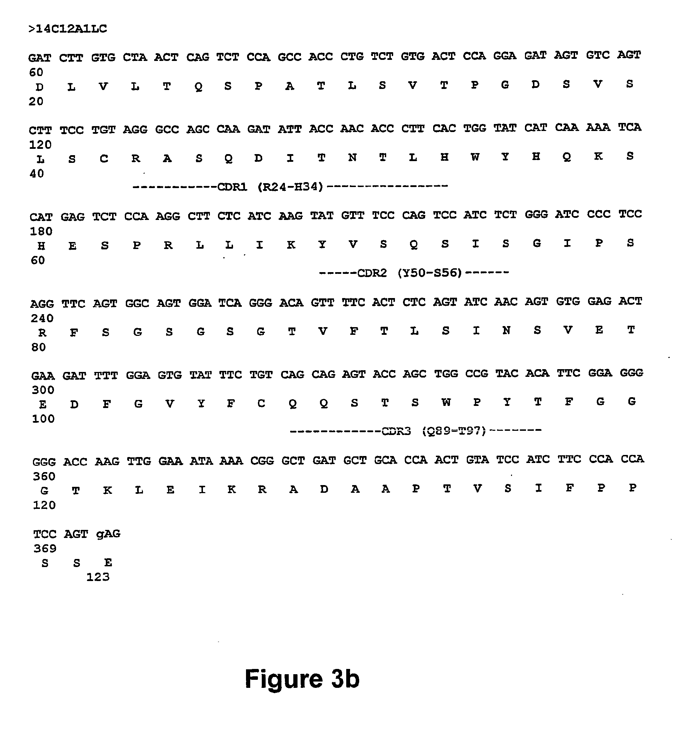

A thorough biochemical evaluation of Ab 14C12 was carried out. Ab 14C12 binds with high affinity to B02C11, with kₒₙ and k_{off} values of 10⁵ m⁻¹ S⁻¹, 10⁻⁵ S⁻¹ respectively, as measured using a surface plasmon resonance system. The VH and VL domains of Ab 14C12 were sequenced by conventional methods (figure 3). Thus Ab 14C12 belongs to the IJHV1 heavy chain antibody family, with a kappa light chain. The capacity of Ab 14C12 to inhibit the binding of BO2C11 to the FVIII C2 domain prompted the inventors to compare the sequence of the VH and VL domains of AB14C12 with that of C2. The CDR1 and CDR2 of VH contain 6 and 7 amino acid residues that are identical or homologous to the C2 domain, respectively. CDR2 is almost identical to the V2223-T2237 C2 region, which contains a crucial phospholipid-binding site and a number of contact residues for BO2C11. In addition, based on the sequence of BO2C11 variable parts (Jacquemin MG et al. in (1998) Blood 92: 496-506) and its crystal structure in association with C2 (Spiegel P.C. Jr. et al. in (2001) Blood 98: 13-19), putative contact residues between BO2C11 and Ab 14C12 were identified, some of which are located within the framework of BO2C11 variable parts. Taken together, these data show that the variable part of the heavy chain of Ab 14C12 contains both an internal image of C2 made of 13 identical or homologous amino acid residues, and a number of contact residues for the variable part of BO2C11.

Ab 14C12 therefore represents the first example of an anti-idiotypic antibody directed towards the C2 domain of FVIII, administration of which restores FVIII activity in vivo in a murine model of haemophilia A. As such, it discloses an effective therapy for haemophilia A patients with FVIII inhibitors.

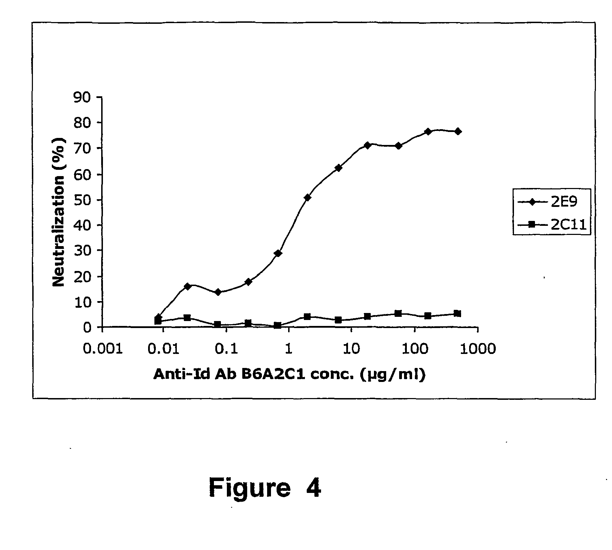

To determine whether another anti-idiotypic antibody against an anti-FVIII C1 domain inhibitor, named B6A2C1 (Gilles JG et al in (1999) Blood 94, abstract 2048, 460a), can interfere with the binding of BO2C11 to FVIII, serial dilutions of this antibody were made and tested for their capacity to inhibit the binding of B02C11 to FVIII-coated polystyrene plates. Figure 4 shows that no inhibition occurred, indicating that B6A2C1 expressed no specificity for BO2C11.

### EXAMPLE 5: Analysis of the homology between Ab14C12 and the FVIII C2 domain

The in vitro and in vivo properties of the anti-idiotypic antibody (Ab 14C12) described in examples 2 and 3, respectively (see above), suggested that an extensive homology exists between the variable parts of the anti-idiotypic antibody and the C2 domain of FVIII. The crystal structure of the C2 domain has been obtained (Pratt K et al. in (1999) Nature 402: 439-442). The sequences of the variable parts of the heavy and light chain of Ab 14C12 were therefore aligned to those of the C2 domain.

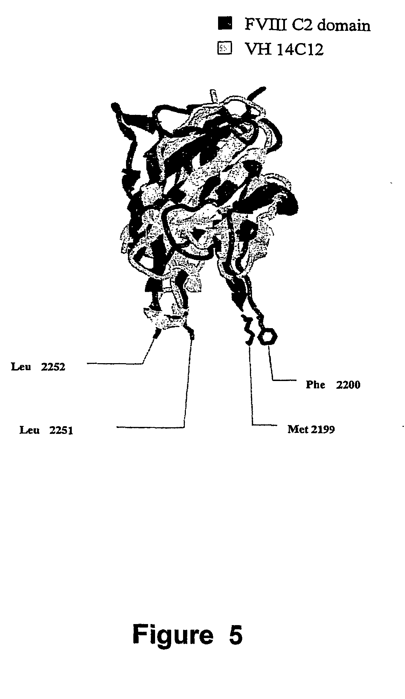

A significant homology was found with the VH region. Table 1 shows that 31 aminoacid residues were found either identical or similar. This included 13 residues associated with CDR regions, but clustering to the CDR1 (H1-16 residues) and CDR3 (H3- 6 residues). Interestingly, a significant contribution is observed from residues located in framework regions (18 aminoacids). Moreover, only two residues (marked with an asterisk) of the VH region are mutated from the germline sequence of Ab14C12. All 31 residues are surface-exposed on C2, according to the crystal structure of the C2 domain (Pratt K et al. in (1999) Nature 402: 439-442), as well as on Ab14C12 VH region. A salient feature of this alignment is that it indicates the involvement of one of the two C2 phospholipid binding sites made up of Leu2251 and Leu2252 (total surface of 180 A), which correspond to Leu102 and Leu103 in Ab14C12 VH CDR3, while the second site (Met2199 and Phe2200) is not represented. Taken together these observations indicate that the VH region of Ab14C12 in its germline configuration carries an extended homology to the C2 domain. Figure 5 illustrates the superposition of the C2 domain with Ab 14C12 VH region.

Another important aspect of the information provided in Table 1 is that the aminoacid residues of Ab 14C12 VH that mimic surface residues of the C2 domain are not part of a continuous sequence, but rather spread over the entire VH (from residues S7 to S121). This finding is in keeping with the demonstration that inhibitors to the C2 domain of FVIII bind to conformational epitopes rather than to sequential ones (see for instance the crystal structure of BO2C11 and the C2 domain in Spiegel P et al. (2001) Blood 98: 13-19).

### EXAMPLE 6: Determination of the interacting regions between B02C11 and Ab14C12

The specificity of an antibody for a given antigen is generally dependent on residues located in one or two of the complementarity-determining regions (CDRs), often located in the VH region. Binding occurs corresponding to the most favourable thermodynamic conditions (Manivel et al. (2000) Immunity 13:611-620). Upon subsequent exposure to antigen, the antibody response is said to mature, a process which involves the random introduction of mutations in VH and VL regions. This is followed by a selection based on avidity for antigen. Therefore, the binding avidity of fully mature antibodies (which are the ones that can be detected or isolated from the natural repertoire of patients' B cells, in the present case towards FVIII) is dependent on cooperative forces brought about by residues located throughout entire VH and VL regions.

To determine whether the interaction between BO2C11 and Ab14C12 depended on residues located on a small number of discrete regions within the VH region, or, to the contrary, depended on residues scattered over the entire VH and VL regions, a sequence alignment was carried out between the VH and VL regions of BO2C11 and VH and VL regions of Ab 14C12. The results shown in Table 2 indicate that a significant contribution to mutual interaction is brought about by residues spread over VH and VL regions of both antibodies. This data confirms that a preferred way to obtain anti-idiotypic antibodies for administration in patients with FVIII inhibitors is to use complete anti-FVIII antibodies.

### EXAMPLE 7: Neutralization of BO2C11 inhibitory activity by Fab fragments of Ab 14C12

Fab fragments of Ab 14C12 were prepared by digestion with papain agarose beads (Pierce, Rockford, IL) and purified by passage over a protein-A Sepharose column, according to methods well known to those skilled in the art.

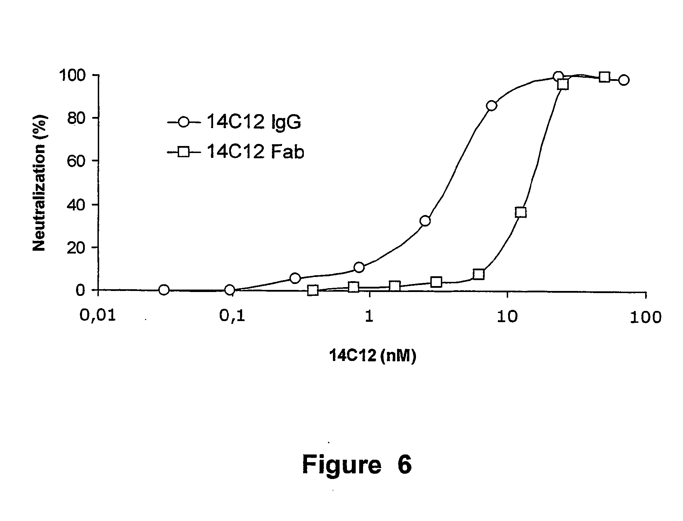

To evaluate the capacity of Ab 14C12 Fab fragments to restore FVIII function in the presence of the inhibitor BO2C11, we first determined the concentration of BO2C11 required to inhibit 80% of FVIII activity in a functional chromogenic assay (Faktor VIII chromogen test, Dade Behring, Marburg, Germany) using 1 IU/ml of recombinant FVIII, as described in Example 2 (see above). The amount of BO2C11 required to inhibit 80% of FVIII activity was mixed with an equal volume of various concentrations of Ab 14C12 Fab fragments. The mixture was incubated for 1 h at 37°C before addition of recombinant FVIII. An aliquot of the mixture was retrieved after a further 1-h incubation at 37°C and added to the chromogenic assay reagents. Control experiments included recombinant FVIII incubated alone or with an Ab of unrelated specificity. Figure 6 shows that Fab fragments neutralize 50% of BO2C11 inhibitory activity at a 1/8 BO2C11/Ab 14C12Fab molar ratio.

### EXAMPLE 8: Influence of Ab 14C12 on binding of FVIII to vWF and PL.

The FVIII C2 domain contains the binding site for phospholipids (PL) and major binding sites for von Willebrand Factor (vWF). Binding to PL is essential for the physiological activity of FVIII, namely the formation of the tenase complex with FIX and FX. vWF acts as a chaperone protein, protecting FVIII from early degradation and clearance.

The capacity of Ab 14C12 to inhibit the binding of FVIII to either VWF or PL was investigated as follows. Microtitration plates were coated with an anti-vWF antibody followed by purified vWF as described elsewhere (Jacquemin M et al. (1998) Blood 95: 156-163). Dilutions of Ab 14C12 (from 100 to 0.3 µg/ml) were prepared and an aliquot of each dilution was added to plates coated with vWF, followed by addition of FVIII at a final concentration of 1 IU/ml. Plates were incubated for 2 hours at room temperature. The binding of FVIII to vWF-coated plates was detected by addition of 2 µg/ml of biotin-labeled mAb15 (an anti-FVIII antibody recognizing a distant site of FVIII located on the heavy chain), followed by avidin-peroxidase and a specific substrate. To assess FVIII binding on PL, plates were coated with phosphathidylserine diluted at 10 µg/ml in methanol and the assay carried out as for the vWF-coated plates.

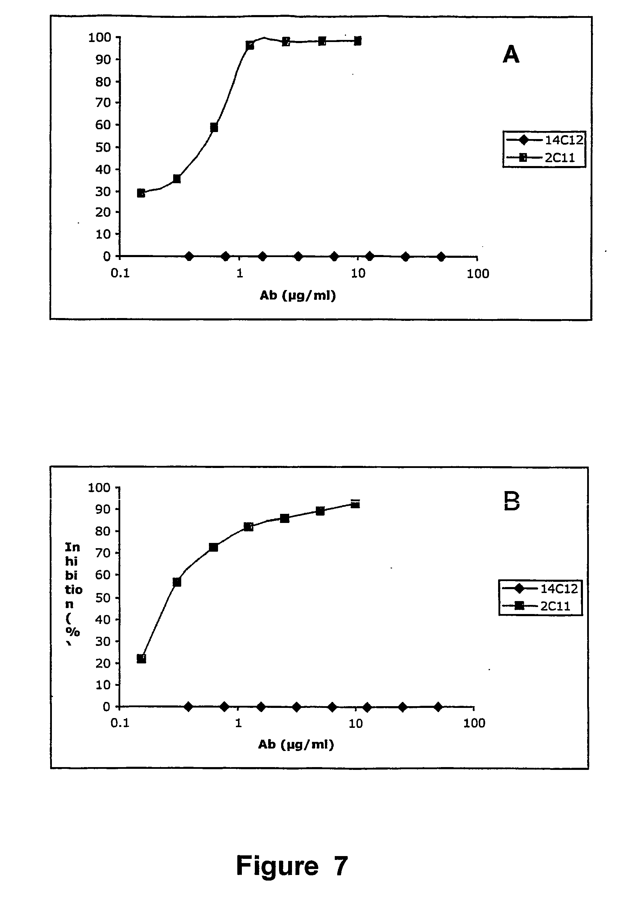

No inhibition of FVIII binding (and therefore of the C2 domain) to either vWF or PL was observed (Figure 7). A control experiment in which Ab 14C12 was replaced by BO2C11 showed complete inhibition of FVIII binding to both vWF- and PL-coated plates (data not shown; see Figure 3 in Jacquemin M et al. (1998) Blood 95: 156-163). It is therefore concluded that the extensive homology between the C2 domain and Ab 14C12 VH is not sufficient to interfere in the binding of FVIII to PL and/or VWF. Administration of Ab 14C12, or of one of its derivatives, as a mode of therapy for patients having inhibitors to the C2 domain should therefore not result in undesirable inhibition of FVIII functional properties.

### EXAMPLE 9: Influence of Ab 14C12 on the clearance of FVIII

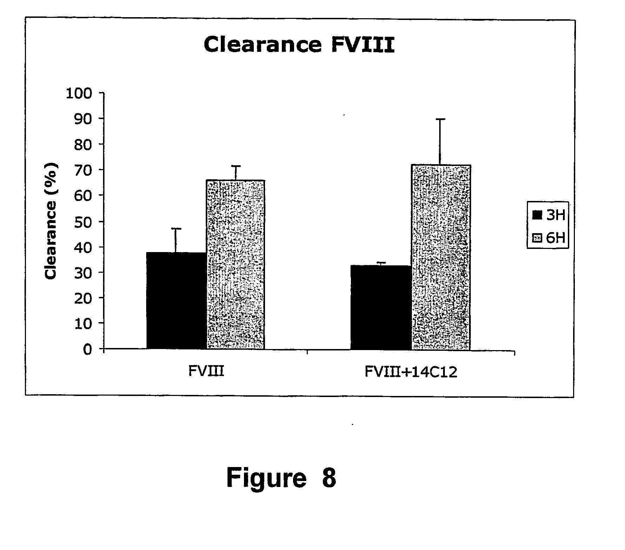

The clearance of FVIII from the circulation is at least in part due to the binding of the C2 domain to the LRP receptor (Saenko et al. (1999) J Biol Chem 274: 37685-37692; Lenting et al. (1999) J Biol Chem 274: 23734-23739) and could therefore be reduced by Ab 14C12. This was examined in FVIII-/- C57BU6 mice reconstituted by tail injection of 1 IU human recombinant FVIII. Previous calculations have shown that the average human FVIII t_{½} in this model was of 165 min. To ensure that Ab 14C12 did not modify FVIII clearance, . C57BU6 FVIII-/- mice were injected with 10 µg/ml of Ab 14C12 or of an IgG2a monoclonal antibody of unrelated specificity, followed 15 min later by an IV injection of 1 IU of recombinant FVIII. Blood samples were collected by venipuncture after 3 and 6 hours. Residual FVIII activity was evaluated by using a functional chromogenic assay (Faktor VIII chromogen test, Dade Behring, Marburg, Germany). No significant difference in FVIII t_{½} was observed in the presence of Ab 14C12, as shown in Figure 8. Administration of Ab 14C12, or of one of its derivatives, as a mode of therapy for patients having inhibitors to the C2 domain should therefore not alter the physiological clearance of FVIII.

### EXAMPLE 10: Binding of Ab 14C12 to BO2C11-producing B-cell line.

Ab 14C12 can be used to target memory B cells and some B cell lines producing producing antibodies inhibiting FVIII function. Such B lymphocytes express at their surface an antibody molecule with variable regions identical to those of the secreted form of the antibody. The binding of Ab 14C12 to its target cells can be used to transduce a signal that would result in cell death, or apoptosis, thereby purging the immune system from cells producing anti-FVIII inhibitors in an exquisitely specific manner.

The BO2C11 producing B-cell line (Jacquemin M et al. (1998) Blood 95: 156-163) produces the BO2C11 soluble antibody and expresses the antibody variable parts on its surface. To determine whether Ab 14C12 could bind to cell the cell surface, the BO2C11 cell line was washed in a buffer containing 0.5% bovine serum albumin and 2mM EDTA, centrifuged at 400g during 5 min and resuspended in the same buffer at a concentration of 5x10⁵ cells in 100 µl. Antibodies, either Ab 14C12 or a sham IgG2a antibody are then added to the cell suspension, using two different concentrations, either 1 or 10 µg/ml. The mixtures are incubated for 20 minutes on ice, washed by centrifugation and resuspension in buffer as described above. An anti-mouse IgG antibody coupled to FITC is then added to each cell suspension at a final concentration of 1 µg/ml for a further incubation on ice for 20 minutes. Cell suspensions are finally washed by centrifugation and resuspension in buffer as above. The final suspension was made to obtain a cell concentration of 1x10⁵ in 500 µl for counting in a fluorescence-activated cell sorter.

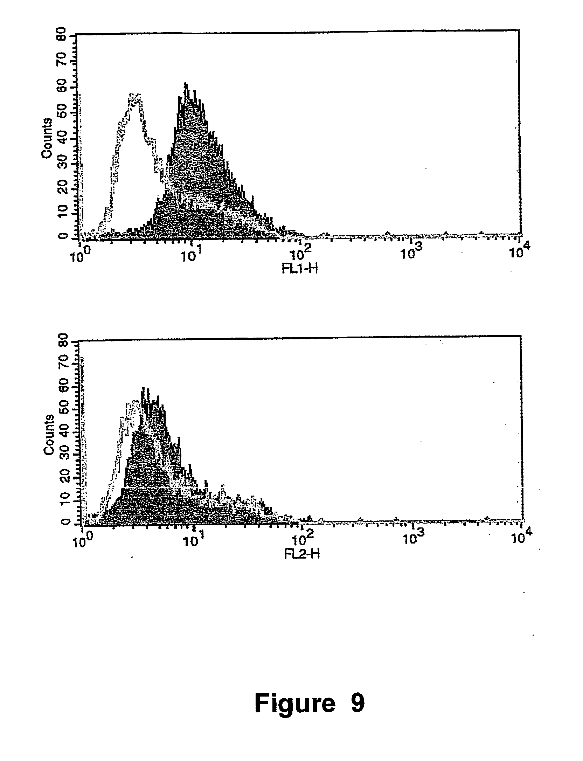

Figure 9 shows that after incubation with Ab 14C12, the BO2C11 cell line was specifically labeled with Ab 14C12, with 68 % of cells positive for the antibody, while a sham antibody of the same subclass did not show any reactivity.

Thus, Ab 14C12 can be used as a specific reagent to target cells producing antibodies identical or similar to BO2C11, thereby providing a method for induction of apoptosis in a highly specific context.

**Table 1**

| | VH 14C12 | | C2 domain |
|---|---|---|---|
| FW1 | S7 | = | S2175 |
| | P9 | = | P2177 |
| | V12 | ± | M2180 |
| | P14 | ± | S2182 |
| | A16 | = | A2184 |
| CDR1 | A24 | = | A2192 |
| | S25 | = | S2193 |
| | G26 | ± | S2194 |
| | Y27 | = | Y2195 |
| | F29 | = | F2196© |
| | T30 | = | T2197© |
| FW2 | W36 | = | W2203 |
| | K40 | = | K2207 |
| | Q43 | ± | L2210 |
| | L45 | = | L2212 |
| | W47 | ± | G2214 |
| CDR2 | D56 | ± | V2223© |
| FW3 | S77 | ± | P2226 |
| | M81 | ± | L2230 |
| | *E82 | ± | Q2231 |
| | L83 | ± | V2232 |
| | T87 | = | T2237 |
| | *A92 | ± | T2241 |
| CDR3 | G101 | ± | S2250© |
| | L102 | = | L2251© |
| | L103 | = | L2252© |
| | G105 | ± | S2254 |
| | D110 | ± | E2259 |
| | V111 | = | V2293 |
| FW4 | V120 | ± | L2302 |
| | S121 | ± | T2303 |

| | | | |
|---|---|---|---|
| Table 1: Sequence alignment between Ab 14C12 VH and FVIII C2 domain: Identical and homologous residues are indicated by "=" and "±", respectively. mAb14C12 mutated VH residues are shown with an asterisk. Putative C2 residues in contact with mAb2C11 are indicated by ©. CDR: complementarity determining region; FW: framework; | | | |

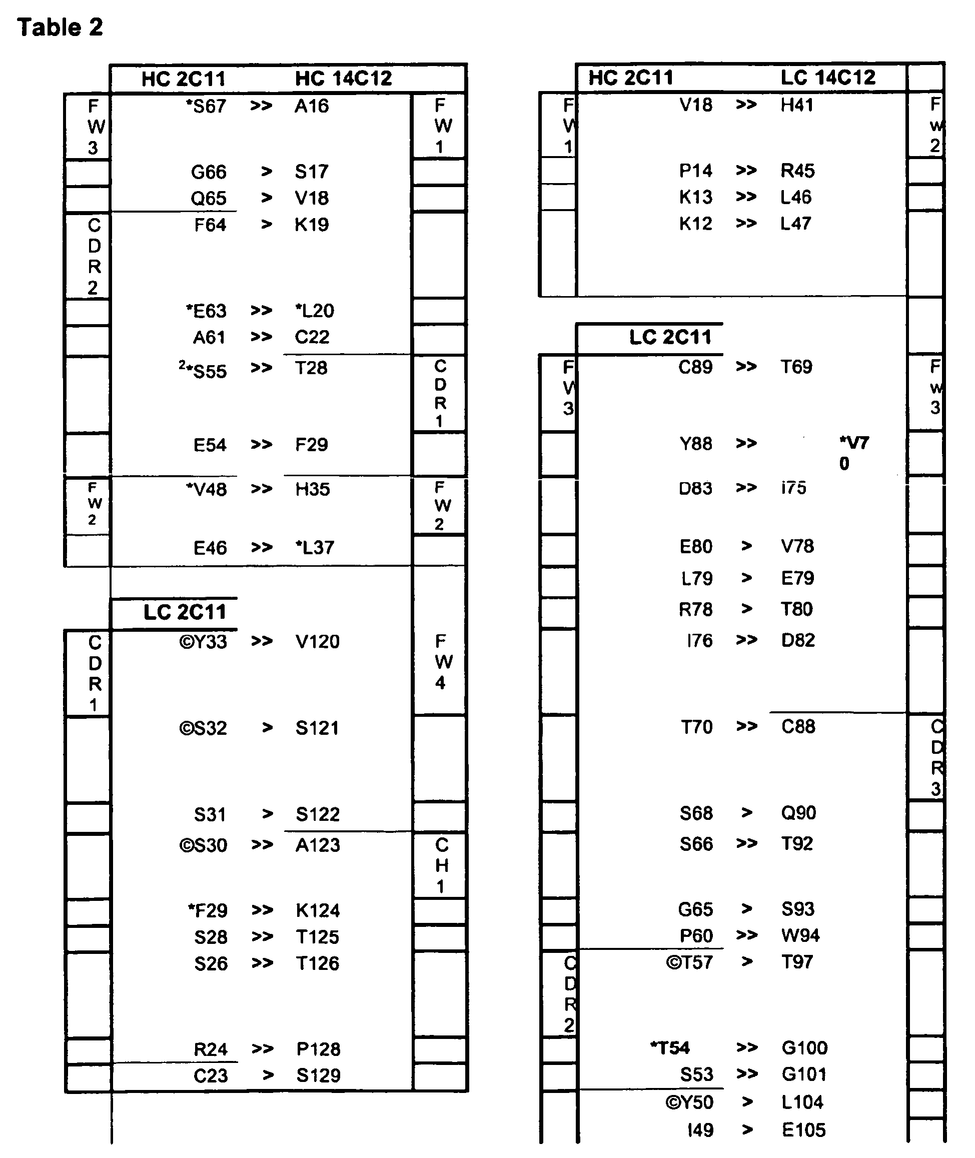

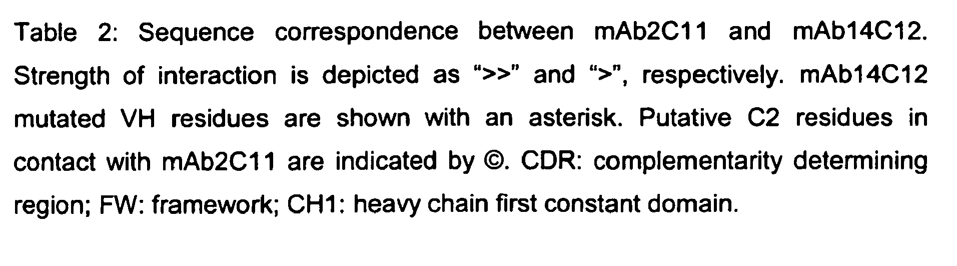

## Claims

1. A monoclonal anti-idiotypic antibody against a human Factor VIII inhibitory antibody, said inhibitory antibody being directed towards the C2 domain of Factor VIII, **characterized by** the fact that the complementary determining regions of the variable heavy chains of said anti-idiotypic antibody have the amino acid sequences depicted in SEQ ID NO:5, SEQ ID NO:6, and SEQ ID NO:7, and the complementary determining regions of the variable light chains of said anti-idiotypic antibody have the amino acid sequences depicted in SEQ ID NO:8 SEQ ID NO:9 and SEQ ID NO:10, and by the fact that the anti-idiotypic antibody a) neutralises the anti-coagulant activity of said human Factor VIII inhibitory antibody for at least 50% in a chromogenic FVIII assay and b) does not interfere with the binding of FVIII to vWF and phospholipids.

2. The monoclonal anti-idiotypic antibody according to claim 1, wherein the variable heavy chain of said anti-idiotypic antibody is encoded by the nucleotide sequence depicted in SEQ ID NO: 1 or a nucleotide sequence having at least 95% sequence identity to SEQ ID NO: 1 or wherein the variable light chain of the anti-idiotypic antibody is encoded by the nucleotide sequence depicted in SEQ ID NO: 3 or a nucleotide sequence having at least 95% sequence identity with SEQ ID NO: 3.

3. The monoclonal anti-idiotypic antibody according to claim 1 , wherein the variable heavy chain of said anti-idiotypic antibody is encoded by the nucleotide sequence depicted in SEQ ID NO: 1 or a nucleotide sequence having at least 70% sequence identity to SEQ ID NO: 1 and wherein the variable light chain of the anti-idiotypic antibody is encoded by the nucleotide sequence depicted in SEQ ID NO: 3 or a nucleotide sequence having at least 70% sequence identity with SEQ ID NO: 3.

4. The monoclonal anti-idiotypic antibody according to any of claims 1 to 3, which is an F(Ab')2 fragment, an Fab' fragment, an Fab fragment or a modified version of said fragment.

5. The monoclonal anti-idiotypic antibody according to any of claims 1 to 4, which is a humanized monoclonal anti-idiotypic antibody.

6. The monoclonal anti-idiotypic antibody according to any of claims 1 to 5, which is 14C12 produced by cell line 14C12 deposited at BCCM with Accession Number LMBP 5878CB or an antibody fragment therefrom containing the antibody binding site.

7. A monoclonal cell line expressing a monoclonal anti-idiotypic antibody in accordance with any of claims 1 to 6.

8. The monoclonal cell line in accordance with claim 7, which is the cell line 14C12 deposited at BCCM with Accession Number LMBP 5878CB.

9. A pharmaceutical composition comprising a monoclonal anti-idiotypic antibody according to any of claims 1 to 6, in admixture with at least one pharmaceutically acceptable carrier.

10. Use of a monoclonal anti-idiotypic antibody according to any one of claims 1 to 6 for the preparation of a medicament for the treatment of a hemophilia patient suffering from bleeding, or for the prevention of bleeding in said patient.

11. Use of a monoclonal anti-idiotypic antibody according to any one of claims 1 to 6 for the preparation of a medicament for the treatment or prevention of uncontrolled bleeding in a hemophilia patient with FVIII inhibitory antibodies.

12. The pharmaceutical composition according to claim 9, which further comprises FVIII.

13. A method for developing monoclonal anti-idiotypic antibodies for the manufacture of a medicament against FVIII inhibitors, said method comprising immunizing a non-human animal with inhibitory antibodies directed against the C2 domain of FVIII and screening the immortalized spleen cells of said animal for the production of antibodies which a) neutralise the anti-coagulant activity of said inhibitory antibodies for at least 50% in a chromogenic FVIII assay and b) do not interfere with the binding of FVIII to vWF and phospholipids.

14. Use of the anti-idiotypic antibodies of any one of claims 1 to 6, for the in vitro detection or purification of inhibitory FVIII antibodies.

## Revendications

1. Anticorps anti-idiotypique monoclonal contre un anticorps inhibiteur de Facteur VIII humain, ledit anticorps inhibiteur étant dirigé vers le domaine C2 de Facteur VIII, **caractérisé par le fait que** les régions de détermination complémentaires des chaînes lourdes variables dudit anticorps anti-idiotypique ont les séquences d'acides aminés décrites dans SEQ ID n° 5, SEQ ID n° 6 et SEQ ID n° 7, et les régions de détermination complémentaires des chaînes légères variables dudit anticorps anti-idiotypique ont les séquences d'acides aminés décrites dans SEQ ID n° 8, SEQ ID n° 9 et SEQ ID n° 10, et **par le fait que** l'anticorps anti-idiotypique a) neutralise l'activité anticoagulante dudit anticorps inhibiteur de Facteur VIII humain pour au moins 50 % dans un dosage de FVIII chromogène et b) n'interfère pas avec la liaison de FVIII à vWF et à des phospholipides.

2. Anticorps anti-idiotypique monoclonal selon la revendication 1, dans lequel la chaîne lourde variable dudit anticorps anti-idiotypique est codée par la séquence de nucléotides décrite dans SEQ ID n° 1 ou une séquence de nucléotides ayant une identité de séquence d'au moins 95 % avec SEQ ID n° 1 ou dans lequel la chaîne légère variable de l'anticorps anti-idiotypique est codée par la séquence de nucléotides décrite dans SEQ ID n° 3 ou une séquence de nucléotides ayant une identité de séquence d'au moins 95 % avec SEQ ID n° 3.

3. Anticorps anti-idiotypique monoclonal selon la revendication 1, dans lequel la chaîne lourde variable dudit anticorps anti-idiotypique est codée par la séquence de nucléotides décrite dans SEQ ID n° 1 ou une séquence de nucléotides ayant une identité de séquence d'au moins 70 % avec SEQ ID n° 1 et dans lequel la chaîne légère variable de l'anticorps anti-idiotypique est codée par la séquence de nucléotides décrite dans SEQ ID n° 3 ou une séquence de nucléotides ayant une identité de séquence d'au moins 70 % avec SEQ ID n° 3.

4. Anticorps anti-idiotypique monoclonal selon l'une quelconque des revendications 1 à 3, qui est un fragment F(Ab')2, un fragment Fab', un fragment Fab ou une version modifiée dudit fragment.

5. Anticorps anti-idiotypique monoclonal selon l'une quelconque des revendications 1 à 4, qui est un anticorps anti-idiotypique monoclonal humanisé.

6. Anticorps anti-idiotypique monoclonal selon l'une quelconque des revendications 1 à 5, qui est un 14C12 produit par une lignée cellulaire 14C12 déposée au BCCM avec le Numéro Matricule LMBP 5878CB ou un fragment d'anticorps de ce dernier contenant le site de liaison d'anticorps.

7. Lignée cellulaire monoclonale exprimant un anticorps anti-idiotypique monoclonal selon l'une quelconque des revendications 1 à 6.

8. Lignée cellulaire monoclonale selon la revendication 7, qui est la lignée cellulaire 14C12 déposée au BCCM avec le Numéro Matricule LMBP 5878CB.

9. Composition pharmaceutique comprenant un anticorps anti-idiotypique monoclonal selon l'une quelconque des revendications 1 à 6, en mélange avec au moins un vecteur acceptable d'un point de vue pharmaceutique.

10. Utilisation d'un anticorps anti-idiotypique monoclonal selon l'une quelconque des revendications 1 à 6, pour la préparation d'un médicament pour le traitement d'un patient hémophile souffrant de saignement, ou pour la prévention de saignement chez ledit patient.

11. Utilisation d'un anticorps anti-idiotypique monoclonal selon l'une quelconque des revendications 1 à 6, pour la préparation d'un médicament pour le traitement ou la prévention de saignement non contrôlé chez un patient hémophile avec des anticorps inhibiteurs de FVIII.

12. Composition pharmaceutique selon la revendication 9, qui comprend en outre un FVIII.

13. Procédé pour développer des anticorps anti-idiotypiques monoclonaux pour la fabrication d'un médicament contre des inhibiteurs de FVIII, ledit procédé comprenant l'immunisation d'un animal non humain avec des anticorps inhibiteurs dirigés contre le domaine C2 de FVIII et le filtrage des cellules de rate immortalisées dudit animal pour la production d'anticorps qui a) neutralisent l'activité anticoagulante desdits anticorps inhibiteurs pour au moins 50 % dans un dosage de FVIII chromogène et b) n'interfèrent pas avec la liaison de FVIII à vWF et à des phospholipides.

14. Utilisation des anticorps anti-idiotypiques selon l'une quelconque des revendications 1 à 6, pour la détection ou la purification *in vitro* d'anticorps inhibiteurs de FVIII.

## Patentansprüche

1. Monoklonaler anti-idiotypischer Antikörper gegen einen Human-Faktor VIII inhibitorischen Antikörper, wobei der inhibitorische Antikörper zu der C2 Domäne von Faktor VIII gerichtet ist, **gekennzeichnet durch** die Tatsache, dass die Komplementarität bestimmenden Regionen der variablen schweren Ketten des anti-idiotypischen Antikörpers die Aminosäuresequenzen aufweisen, die in SEQ ID NR: 5, SEQ ID NR: 6 und SEQ ID NR: 7 dargestellt sind, und die Komplementarität bestimmenden Regionen der variablen leichten Ketten des anti-idiotypischen Antikörpers die Aminosäuresequenzen aufweisen, die in SEQ ID NR: 8, SEQ ID NR: 9 und SEQ ID NR: 10 dargestellt sind, und **durch** die Tatsache, dass der anti-idiotypische Antikörper a) die gerinnungshemmende Aktivität des Human-Faktor VIII inhibitorischen Antikörpers um mindestens 50% in einem chromogenen FVIII Assay neutralisiert und b) die Bindung von FVIII an vWF und Phospholipide nicht stört.

2. Monoklonaler anti-idiotypischer Antikörper nach Anspruch 1, wobei die variable schwere Kette des anti-idiotypischen Antikörpers durch die Nukleotidsequenz kodiert wird, die in SEQ ID NR: 1 dargestellt ist, oder eine Nukleotidsequenz mit mindestens 95% Sequenzidentität mit SEQ ID NR: 1, oder wobei die variable leichte Kette des anti-idiotypischen Antikörpers durch die Nukleotidsequenz kodiert wird, die in SEQ ID NR: 3 dargestellt ist, oder eine Nukleotidsequenz mit mindestens 95% Sequenzidentität mit SEQ ID NR: 3.

3. Monoklonaler anti-idiotypischer Antikörper nach Anspruch 1, wobei die variable schwere Kette des anti-idiotypischen Antikörpers durch die Nukleotidsequenz kodiert wird, die in SEQ ID NR: 1 dargestellt ist, oder eine Nukleotidsequenz mit mindestens 70% Sequenzidentität mit SEQ ID NR: 1, und wobei die variable leichte Kette des anti-idiotypischen Antikörpers durch die Nukleotidsequenz kodiert wird, die in SEQ ID NR: 3 dargestellt ist, oder eine Nukleotidsequenz mit mindestens 70% Sequenzidentität mit SEQ ID NR: 3.

4. Monoklonaler anti-idiotypischer Antikörper nach einem der Ansprüche 1 bis 3, der ein F(Ab')2 Fragment, ein Fab' Fragment, ein Fab Fragment oder eine modifizierte Version des Fragments ist.

5. Monoklonaler anti-idiotypischer Antikörper nach einem der Ansprüche 1 bis 4, der ein humanisierter monoklonaler anti-idiotypischer Antikörper ist.

6. Monoklonaler anti-idiotypischer Antikörper nach einem der Ansprüche 1 bis 5, der 14C12 ist, produziert durch Zelllinie 14C12, die bei BCCM mit der Zugriffsnummer LMBP 5878CB hinterlegt ist, oder ein Antikörperfragment davon, das die Antikörperbindungsstelle enthält.

7. Monoklonale Zelllinie, die einen monoklonalen anti-idiotypischen Antikörper nach einem der Ansprüche 1 bis 6 exprimiert.

8. Monoklonale Zelllinie nach Anspruch 7, die die Zelllinie 14C12 ist, die bei BCCM mit der Zugriffsnummer LMBP 5878CB hinterlegt ist.

9. Pharmazeutische Zusammensetzung, umfassend einen monoklonalen anti-idiotypischen Antikörper nach einem der Ansprüche 1 bis 6 in Beimischung mit mindestens einem pharmazeutisch annehmbaren Träger.

10. Verwendung eines monoklonalen anti-idiotypischen Antikörpers nach einem der Ansprüche 1 bis 6 für die Zubereitung eines Medikaments für die Behandlung eines Hämophilie-Patienten, der an Blutungen leidet, oder für die Prävention einer Blutung in dem Patienten.

11. Verwendung eines monoklonalen anti-idiotypischen Antikörpers nach einem der Ansprüche 1 bis 6 für die Zubereitung eines Medikaments für die Behandlung oder Prävention einer unkontrollierten Blutung in einem Hämophilie-Patienten mit FVIII inhibitorischen Antikörpern.

12. Pharmazeutische Zusammensetzung nach Anspruch 9, die des Weiteren FVIII umfasst.

13. Verfahren zur Entwicklung monoklonaler anti-idiotypischer Antikörper für die Herstellung eines Medikaments gegen FVIII-Inhibitoren, wobei das Verfahren das Immunisieren eines nicht-humanen Tiers mit inhibitorischen Antikörpern, die gegen die C2 Domäne von FVIII gerichtet sind, und das Screenen der immortalisierten Milzzellen des Tiers auf die Produktion von Antikörpern umfasst, die a) die gerinnungshemmende Aktivität der inhibitorischen Antikörper um mindestens 50% in einem chromogenen FVIII Assay neutralisieren und b) die Bindung von FVIII an vWF und Phospholidpie nicht stören.

14. Verwendung der anti-idiotypischen Antikörper nach einem der Ansprüche 1 bis 6 für den In-vitro-Nachweis oder die Reinigung inhibitorischer FVIII Antikörper.


### SEQUENCE LISTING

<110> D. Collen Research Foundation vzw
   Gilles, Jean Guy G
   Jacquemin, Marc
   Saint-Remy, Jean-Marie R
<120> Anti-idiotypic antibodies against Factor VIII inhibitor and uses thereof
<130> C 2104 EP
<160> 10
<170> PatentIn version 3.1
<210> 1
   <211> 411
   <212> DNA
   <213> Mus musculus
<220>
   <221> CDS
   <222> (1) .. (411)
   <223>
<220>
   <221> misc_feature
   <222> (78)..(111)
   <223> CDR1
<220>
   <221> misc_feature
   <222> (150)..(198)
   <223> CDR2
<220>
   <221> misc_feature
   <222> (297)..(333)
   <223> CDR2
<220>
   <221> V_region
   <222> (1)..(411)
   <223> 14C12 monoclonal antibody heavy chain
<400> 1
   gag gtc cag ctt cag cag tct gga cct gag ctg gtt aag cct ggg gct      48
   Glu Val Gln Leu Gln Gln Ser Gly Pro Glu Leu Val Lys Pro Gly Ala
   1               5                   10                  15
   tca gtg aag ctg tcc tgc aag gct tct gga tac aca ttc act agc tct      96
   Ser Val Lys Leu Ser Cys Lys Ala Ser Gly Tyr Thr Phe Thr Ser Ser
               20                  25                  30
   gtt atg cac tgg ctg aag cag aag tct ggg cag ggc ctt gag tgg att     144
   Val Met His Trp Leu Lys Gln Lys Ser Gly Gln Gly Leu Glu Trp Ile
           35                  40                  45
   gga tat att aat cct tac aat gat ggt act aag tac aat gag aag ttc     192
   Gly Tyr Ile Asn Pro Tyr Asn Asp Gly Thr Lys Tyr Asn Glu Lys Phe
       50                  55                  60
   aca gcc aag gcc aca ctg act tca gac aaa tcc tcc agc aca gtc tac     240
   Thr Ala Lys Ala Thr Leu Thr Ser Asp Lys Ser Ser Ser Thr Val Tyr
   65                  70                  75                  80
   atg gag ctc agc ggc ctg acc tct gag gac ttt gcg gtc tat tac tgt     288
   Met Glu Leu Ser Gly Leu Thr Ser Glu Asp Phe Ala Val Tyr Tyr Cys
                   85                  90                  95
   gca cga tcg gga ggt tta cta cga ggt tac tgg tac ttc gat gtc tgg     336
   Ala Arg Ser Gly Gly Leu Leu Arg Gly Tyr Trp Tyr Phe Asp Val Trp
               100                 105                 110
   ggc gca ggg acc acg gtc acc gtc tcc tca gcc aaa aca aca gcc cca     384
   Gly Ala Gly Thr Thr Val Thr Val Ser Ser Ala Lys Thr Thr Ala Pro
           115                 120                 125
   tcg gtc tat ccc ttg gtc cct ggc tgc                                 411
   Ser Val Tyr Pro Leu Val Pro Gly Cys
       130                 135
<210> 2
   <211> 137
   <212> PRT
   <213> Mus musculus
<220>
   <221> misc_feature
   <222> (78)..(111)
   <223> CDR1
<220>
   <221> misc_feature
   <222> (150)..(198)
   <223> CDR2
<220>
   <221> misc_feature
   <222> (297)..(333)
   <223> CDR2
<400> 2
   Glu Val Gln Leu Gln Gln Ser Gly Pro Glu Leu Val Lys Pro Gly Ala
   1 5 10 15
   Ser Val Lys Leu Ser Cys Lys Ala Ser Gly Tyr Thr Phe Thr Ser Ser
   20 25 30
   Val Met His Trp Leu Lys Gln Lys Ser Gly Gln Gly Leu Glu Trp Ile
   35 40 45
   Gly Tyr Ile Asn Pro Tyr Asn Asp Gly Thr Lys Tyr Asn Glu Lys Phe
   50 55 60
   Thr Ala Lys Ala Thr Leu Thr Ser Asp Lys Ser Ser Ser Thr Val Tyr
   65 70 75 80
   Met Glu Leu Ser Gly Leu Thr Ser Glu Asp Phe Ala Val Tyr Tyr Cys
   85 90 95
   Ala Arg Ser Gly Gly Leu Leu Arg Gly Tyr Trp Tyr Phe Asp Val Trp
   100 105 110
   Gly Ala Gly Thr Thr Val Thr Val Ser Ser Ala Lys Thr Thr Ala Pro
   115 120 125
   Ser Val Tyr Pro Leu Val Pro Gly Cys
   130 135
<210> 3
   <211> 369
   <212> DNA
   <213> Mus musculus
<220>
   <221> CDS
   <222> (1)..(369) <223>
<220>
   <221> misc_feature
   <222> (72)..(102)
   <223> CDR1
<220>
   <221> misc_feature
   <222> (150)..(168)
   <223> CDR2
<220>
   <221> misc_feature
   <222> (267)..(291)
   <223> CDR3
<220>
   <221> V_region
   <222> (1)..(369)
   <223> 14C12 monoclonal antibody light chain
<400> 3
   gat ctt gtg cta act cag tct cca gcc acc ctg tct gtg act cca gga       48
   Asp Leu Val Leu Thr Gln Ser Pro Ala Thr Leu Ser Val Thr Pro Gly
   1               5                   10                  15
   gat agt gtc agt ctt tcc tgt agg gcc agc caa gat att acc aac acc       96
   Asp Ser Val Ser Leu Ser Cys Arg Ala Ser Gln Asp Ile Thr Asn Thr
               20                  25                  30
   ctt cac tgg tat cat caa aaa tca cat gag tct cca agg ctt ctc atc      144
   Leu His Trp Tyr His Gln Lys Ser His Glu Ser Pro Arg Leu Leu Ile
           35                  40                  45
   aag tat gtt tcc cag tcc atc tct ggg atc ccc tcc agg ttc agt ggc      192
   Lys Tyr Val Ser Gln Ser Ile Ser Gly Ile Pro Ser Arg Phe Ser Gly
       50                  55                  60
   agt gga tca ggg aca gtt ttc act ctc agt atc aac agt gtg gag act      240
   Ser Gly Ser Gly Thr Val Phe Thr Leu Ser Ile Asn Ser Val Glu Thr
   65                  70                  75                  80
   gaa gat ttt gga gtg tat ttc tgt cag cag agt acc agc tgg ccg tac      288
   Glu Asp Phe Gly Val Tyr Phe Cys Gln Gln Ser Thr Ser Trp Pro Tyr
                   85                  90                  95
   aca ttc gga ggg ggg acc aag ttg gaa ata aaa cgg gct gat gct gca      336
   Thr Phe Gly Gly Gly Thr Lys Leu Glu Ile Lys Arg Ala Asp Ala Ala
               100                 105                 110
   cca act gta tcc atc ttc cca cca tcc agt gag                          369
   Pro Thr Val Ser Ile Phe Pro Pro Ser Ser Glu
           115                 120
<210> 4
   <211> 123
   <212> PRT
   <213> Mus musculus
<220>
   <221> misc_feature
   <222> (72)..(102)
   <223> CDR1
<220>
   <221> misc_feature
   <222> (150)..(168)
   <223> CDR2
<220>
   <221> misc_feature
   <222> (267)..(291)
   <223> CDR3
<400> 4
   Asp Leu Val Leu Thr Gln Ser Pro Ala Thr Leu Ser Val Thr Pro Gly
   1               5                   10                  15
   Asp Ser Val Ser Leu Ser Cys Arg Ala Ser Gln Asp Ile Thr Asn Thr
               20                  25                  30
   Leu His Trp Tyr His Gln Lys Ser His Glu Ser Pro Arg Leu Leu Ile
           35                  40                  45
   Lys Tyr Val Ser Gln Ser Ile Ser Gly Ile Pro Ser Arg Phe Ser Gly
       50                  55                  60
   Ser Gly Ser Gly Thr Val Phe Thr Leu Ser Ile Asn Ser Val Glu Thr
   65                  70                  75                  80
   Glu Asp Phe Gly Val Tyr Phe Cys Gln Gln Ser Thr Ser Trp Pro Tyr
                   85                  90                  95
   Thr Phe Gly Gly Gly Thr Lys Leu Glu Ile Lys Arg Ala Asp Ala Ala
               100                 105                 110
   Pro Thr Val Ser Ile Phe Pro Pro Ser Ser Glu
           115                 120
<210> 5
   <211> 12
   <212> PRT
   <213> Mus musculus
<400> 5
   Gly Tyr Thr Phe Thr Ser Ser Val Met His Trp Leu
   1               5                   10
<210> 6
   <211> 18
   <212> PRT
   <213> Mus musculus
<400> 6
   Gly Tyr Ile Asn Pro Tyr Asn Asp Gly Thr Lys Tyr Asn Glu Lys Phe
   1 5 10 15
   Thr Ala
<210> 7
   <211> 13
   <212> PRT
   <213> Mus musculus
<400> 7
   Ser Gly Gly Leu Leu Arg Gly Tyr Trp Tyr Phe Asp Val
   1 5 10
<210> 8
   <211> 11
   <212> PRT
   <213> Mus musculus
<400> 8
   Arg Ala Ser Gln Asp Ile Thr Asn Thr Leu His
   1 5 10
<210> 9
   <211> 7
   <212> PRT
   <213> Mus musculus
<400> 9
   Tyr Val Ser Gln Ser Ile Ser
   1 5
<210> 10
   <211> 9
   <212> PRT
   <213> Mus musculus
<400> 10
   Gln Gln Ser Thr Ser Trp Pro Tyr Thr
   1 5